(19) **Europäisches Patentamt / European Patent Office / Office européen des brevets**

(11) **EP 1 576 226 B1**

(12) **EUROPEAN PATENT SPECIFICATION**

(45) Date of publication and mention
of the grant of the patent:
**03.12.2008 Bulletin 2008/49**

(21) Application number: **03777840.4**

(22) Date of filing: **22.10.2003**

(51) Int Cl.:
*D06M 11/74* (2006.01)    *A61L 15/46* (2006.01)
*A61L 15/18* (2006.01)    *C03C 25/44* (2006.01)
*B01J 20/20* (2006.01)

(86) International application number:
**PCT/US2003/033653**

(87) International publication number:
**WO 2004/061195 (22.07.2004 Gazette 2004/30)**

(54) **FLEXIBLE SUBSTRATES WITH AN ACTIVATED CARBON COATING**

FLEXIBLE SUBSTRATE MIT EINER BESCHICHTUNG AUS AKTIVKOHLE

SUBSTRATS FLEXIBLES PRESENTANT UN REVETEMENT DE CARBONE ACTIVE

(84) Designated Contracting States:
**DE GB**

(30) Priority: **23.12.2002 US 328739**

(43) Date of publication of application:
**21.09.2005 Bulletin 2005/38**

(73) Proprietor: **KIMBERLY-CLARK WORLDWIDE, INC.**
**Neenah WI 54956 (US)**

(72) Inventors:
• **EDENS, Ron**
**Cumming, GA 30041 (US)**
• **GADSBY, Elizabeth, D.**
**Marietta, GA 30068 (US)**

• **LINDSAY, Jeff**
**Appleton, WI 54915 (US)**
• **PIKE, Dan**
**Alpharetta, GA 30004 (US)**
• **MANGUN, Christian**
**Urbana, IL 61802 (US)**

(74) Representative: **Davies, Christopher Robert**
**Frank B. Dehn & Co.**
**St Bride's House**
**10 Salisbury Square**
**London**
**EC4Y 8JD (GB)**

(56) References cited:
**WO-A-01/97972**        **WO-A-01/98224**
**DE-A- 3 339 756**       **US-A- 5 834 114**

EP 1 576 226 B1

**Description**

**Background of the Invention**

While the primary focus of absorbent articles remains the ability of the articles to absorb and retain fluids, additional functions, such as odor control, are also receiving increased attention. A wide range of compounds that result in the production of malodors may be contained in absorbed fluids or their degradation products and thus be present within an absorbent article during use. Examples of these compounds include fatty acids, ammonia, amines, sulfur-containing compounds, ketones and aldehydes. In the past, various odor-control agents have been used in absorbent articles to address the problems of malodor formation. For instance, activated carbon has been used to reduce a broad spectrum of odors. However, conventional activated carbon poses many problems. For instance, it is often difficult to keep loose activated carbon particles in the desired location of the absorbent article. In addition, the particles also generate dust and undesired noise. Thus, activated carbon fabrics were developed with pitch or another material that was activated at high temperatures. Unfortunately, however, these products are generally expensive and brittle.

As such, a need currently exits for an improved activated carbon fibrous material that is also flexible. US-A-5,834,114 discloses industrial filters formed of nonwoven materials with an activated carbon coating.

**Summary of the Invention**

In accordance with the present invention, a flexible substrate is disclosed that is applied with an activated carbon coating, the substrate being embodied in a sanitary napkin, diaper, diaper pant, feminine napkin or child's training pant. The flexible substrate contains a stretchable component that, upon application of a force, is stretchable to a stretched, biased length that is at least about 120% its unstretched length. The stretchable component may include a polymeric mesh, such as polyester meshes, nylon meshes, and combinations thereof. Moreover, in some embodiments, the stretchable component may include an elastomeric polymer. The softening point of the elastomeric polymer may be greater than the activation temperature of the activated carbon coating. Some examples of suitable elastomeric polymers include, but are not limited to, fluoropolymers; perfluoropolymers; highly saturated nitrile polymers; ethylene vinyl acetate polymers, silicone polymers; polyacrylate elastomers; and combinations thereof. The elastomeric polymer may also be contained within an elastic laminate, such as neck-bonded laminates, stretch-bonded laminates, necked stretched-bonded laminates, and combinations thereof. The activated carbon coating is formed from a polymeric material and an activation agent. The coating may become activated by being heated to a temperature of from about 100°C to about 300°C, and in some embodiments, from about 170°C to about 300°C.

The polymeric material may be selected from the group consisting of polyacrylonitrile, phenolic resins, ethylene vinyl acetate or copolymers thereof, polyvinyl alcohol, cellulose, polystyrene, polypropylene, polyvinyl chloride, polymethacrylates, polymethacrylic acids, and combinations thereof. Further, the activation agent may be selected from the group consisting of acids, metal halides, hydroxides, and combinations thereof. In some embodiments, the activated carbon coating is configured to adsorb acidic compounds, basic compounds, or combinations thereof.

The activated carbon coating may generally be applied in a variety of ways. For instance, in one embodiment, the activated carbon coating is applied in a preselected pattern on a first surface of the substrate. If desired, a second activated carbon coating may also be applied to the substrate. In one embodiment, the second activated carbon coating is applied to a second surface of the substrate. The second activated carbon coating may contain a different amount of activated carbon than the first activated carbon coating.

Other features and aspects of the present invention are discussed in greater detail below.

**Brief Description of the Drawings**

A full and enabling disclosure of the present invention, including the best mode thereof, directed to one of ordinary skill in the art, is set forth more particularly in the remainder of the specification, which makes reference to the appended figures in which:

Fig. 1 illustrates a perspective view of a sanitary napkin formed according to one embodiment of the present invention;
Fig. 2 illustrates the fabric formed according to the process set forth in Example 1 without activated carbon treatment;
Fig. 3 illustrates the fabric formed according to the process set forth in Example 1 with activated carbon treatment;
Fig. 4 illustrates an activated carbon fabric formed according to the process set forth in Example 2;
Fig. 5 is a scanned grayscale image of a nonwoven fabric sample treated with two different activated carbon precursor solutions, according to Example 17; and
Fig. 6 is another scanned grayscale image of a nonwoven fabric sample treated with two different activated carbon precursor solutions, according to Example 17.

## Detailed Description of Representative Embodiments

Definitions

As used herein, the term "absorbent article" refers to any article capable of absorbing water or other fluids. Examples of some absorbent articles include, but are not limited to, personal care absorbent articles, such as diapers, training pants, absorbent underpants, adult incontinence products, feminine hygiene products (e.g., sanitary napkins), and the like; wound coverings; wipers; bed pads; shoe pads; clothing articles, such as perspiration pads, disposable swimming apparel, and the like; air and water filtration devices; and the like. Materials and processes suitable for forming such absorbent articles are well known to those skilled in the art.

As used herein the term "nonwoven fabric or web" means a web having a structure of individual fibers or threads which are interlaid, but not in an identifiable manner as in a knitted fabric. Nonwoven fabrics or webs have been formed from many processes such as for example, meltblowing processes, spunbonding processes, bonded carded web processes, needle punching, apertured film production processes, etc.

As used herein, the term "meltblown fibers" refers to fibers formed by extruding a molten thermoplastic material through a plurality of fine, usually circular, die capillaries as molten fibers into converging high velocity gas (e.g. air) streams that attenuate the fibers of molten thermoplastic material to reduce their diameter, which may be to microfiber diameter. Thereafter, the meltblown fibers are carried by the high velocity gas stream and are deposited on a collecting surface to form a web of randomly disbursed meltblown fibers. Such a process is disclosed, for example, in U.S. Pat. No. 3,849,241 to Butin, et al. Generally speaking, meltblown fibers may be microfibers that may be continuous or discontinuous, are generally smaller than 10 microns in diameter, and are generally tacky when deposited onto a collecting surface.

As used herein, the term "spunbonded fibers" refers to small diameter substantially continuous fibers that are formed by extruding a molten thermoplastic material from a plurality of fine, usually circular, capillaries of a spinnerette with the diameter of the extruded fibers then being rapidly reduced as by, for example, eductive drawing and/or other well-known spunbonding mechanisms. The production of spunbonded nonwoven webs is described and illustrated, for example, in U.S. Patent Nos. 4,340,563 to Appel, et al., 3,692,618 to Dorschner, et al., 3,802,817 to Matsuki et al., 3,338,992 to Kinney, 3,341,394 to Kinney, 3,502,763 to Hartman, 3,502,538 to Levy, 3,542,615 to Dobo, et al., and 5,382,400 to Pike, et al. Spunbond fibers are generally not tacky when they are deposited onto a collecting surface. Spunbond fibers can sometimes have diameters less than about 40 microns, and are often between about 5 to about 20 microns.

As used herein, the term "coform" generally refers to composite materials comprising a mixture or stabilized matrix of thermoplastic fibers and a second non-thermoplastic material. As an example, coform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic absorbent materials, treated polymeric staple fibers and the like. Some examples of such coform materials are disclosed in U.S. Patent Nos. 4,100,324 to Anderson, et al.; 5,284,703 to Everhart, et al.; and 5,350,624 to Georger, et al.

Detailed Description

Reference now will be made in detail to various embodiments of the invention, one or more examples of which are set forth below. Each example is provided by way of explanation of the invention, not limitation of the invention. In fact, it will be apparent to those skilled in the art that various modifications and variations can be made in the present invention without departing from the scope of the invention.

In general, the present invention is directed to a flexible substrate applied with an activated carbon coating. The activated carbon coating is formed from a mixture of a polymeric material and an activation agent. The mixture is activated by heating to a temperature of from about 100°C to about 300°C. As a result of the present invention, it has been discovered that an activated carbon-coated substrate can be formed that is flexible and also capable of performing other functions, such as serving as an odor control agent. In addition, because the coating is activated at a relatively low temperature, a wide range of polymers remains available for use in the substrate.

When the activated carbon coating is applied heterogeneously to a substrate, the resulting substrate can sometimes perform better than a homogenous activated carbon substrate. For example, portions of a nonwoven web untreated with activated carbon may be able to allow fluid to flow through the web or be absorbed by the web more effectively than regions treated with activated carbon, while the treated regions remain available to adsorb odors or other chemicals. Thus, a substrate with both activated carbon treated regions and untreated regions can serve effectively for fluid handling purposes (intake or fluid absorption or fluid distribution), while also serving as an odor control layer or adsorption layer. A pattern of treated and untreated regions may also offer more visual appeal than a homogenous activated carbon substrates, such as a pattern of stripes, dots, or other shapes.

When two or more kinds of activated carbon are present on a substrate, according to one embodiment of the present invention, one type may be well suited for adsorbing a certain class of compounds, while the other type(s) may be suited for adsorbing a another class of compounds, such that the heterogeneous activated carbon substrate is effective in adsorbing two or more classes of compounds more effectively than a homogenous activated carbon substrate. For example, activated carbon coatings derived by activation of polyacrylonitrile may be well suited for adsorbing acidic compounds such as isovaleric acid or hydrochloric acid, while activated carbon coatings comprising acidic groups may be effective at adsorbing ammonia or other basic compounds.

A. Activated Carbon Coating

To form the activated carbon substrate, some or all of the substrate is coated with activated carbon. When utilized, for instance, fibers may be coated before and/or after incorporation into the substrate. Generally speaking, the activated carbon coating may be formed in a variety of different ways. One particularly desired method for forming the activated carbon coating is described in U.S. Patent Publication No. 2001/0024716. For instance, in some embodiments, a coating mixture of a polymeric material and a chemical activation agent is applied to fibers that are then heated to induce carbon activation.

The polymeric material of the coating may be any organic polymer that will react with a chemical activation agent to produce an activated carbon coating. Examples of suitable polymeric materials that may be used include, but are not limited to, phenolic resins, ethylene vinyl acetate or copolymers thereof, poly(vinyl alcohol) (PVA), polyacrylonitrile (PAN), cellulose or other natural or synthetic polysaccharides, cellulose derivatives or other polysaccharide derivatives, polystyrene, polypropylene, poly(vinyl chloride) (PVC), poly(meth)acrylates and poly(meth)acrylic acids, polylactic acid, and combinations thereof. Desirably, the polymeric material is soluble in a solvent. Examples of some suitable solvents include, but are not limited to, water; alcohols, such as ethanol or methanol; dimethylformamide (DMF); dimethyl sulfoxide; hydrocarbons, such as pentane, butane, heptane, hexane, toluene and xylene; ethers, such as diethyl ether and tetrahydrofuran; ketones and aldehydes, such as acetone and methyl ethyl ketone; acids, such as acetic acid and formic acid; amines, such as pyridine and hexamethylenetetramine; and halogenated solvents, such as dichloromethane and carbon tetrachloride; and the like.

As stated, the activation agent reacts with the polymeric material to form the activated carbon coating at an elevated temperature. Although not required, Lewis acids and bases may be employed as the activation agents in the present invention. Some examples of such activation agents are described in U.S. Patent No. 5,834,114 to Economy, et al.; WO 01/97972 to Economy, et al.; and U.S. Patent Publication No. 2001/0024716. Specific examples include, but are not limited to, acids, such as phosphoric acid; metal halides, such as zinc chloride; and hydroxides, such as potassium hydroxide and sodium hydroxide. Other examples include Friedel-Crafts compounds; dehydrating agents; $TiC_4$, $ZnBr_2$, $AlBr_3$, $AlCl_3$, $BF_3$, $CaO$, $Ca(OH)_2$, $H_2SO_4$, $Mg(OH)_2$, $MgO$ and $LiOH$.

The amount of the activation agent within the mixture may generally vary as desired. For example, in some embodiments, the activation agent is present in the coating mixture in an amount of from about 0.1 wt.% to about 90 wt%. As the amount of activation agent is increased, the pore size of the resulting activated carbon coating also increases. After reaction has occurred, some or all of the remaining activation agent can be washed out of the activated carbon coating, if desired. For example, substantially all of the remaining activation agent can be removed by washing with water or other substances, or a lesser portion of the remaining activation agent can be removed, such as from about 1% to about 99%, from about 10% to about 99%, or from about 20% to about 99%, or from about 50% to about 95%, or from about 60% to about 95% of the remaining activation agent. In some embodiments, a portion of the activation agent (e.g., a zinc salt or an acidic compound like phosphoric acid or its salts) is left to serve additional functions, such as ion exchange, antimicrobial functions, removal of target species by chemical reaction or neutralization, pH control, viscosity control, surface tension modification, and the like. In such embodiments, the percentage of the initial activation agent or its soluble reaction products that is retained in the activated carbon substrate may be at least about 1%, in some embodiments at least about 10%, in some embodiments at least about 20%, and in some embodiments, at least about 30%.

The activated carbon coating may include one or more catalytic materials that remain inert during processing but catalyze the decomposition of byproduct gases. Examples of suitable catalysts include, but are not limited to, free metals or compounds of metals, such as zinc, copper, platinum, palladium and titanium. In some embodiments, the metal is present as the free metal or the oxide (such as zinc oxide, titanium dioxide, or copper oxide). The catalyst may be applied by mixing it or a compound of the metal of the catalyst into the coating mixture, or after activation by coating the activated carbon coating with a mixture of catalyst, or a compound containing the metal of the catalyst, and a solvent, and then vaporizing the solvent. For example, the metal of the catalyst may be applied as the chloride salt with a solvent, and then heated to remove the solvent and convert the chloride salt to an oxide or the free metal. Any volatile solvent capable of dispersing or dissolving the catalyst or a compound of the metal of the catalyst is suitable, for example water;

alcohols such as ethanol or methanol; dimethylformamide; dimethyl sulfoxide; hydrocarbons such as pentane, butane, heptane, hexane, toluene and xylene; ethers such as diethyl ether and tetrahydrofuran; ketones and aldehydes such as acetone and methyl ethyl ketone; acids such as acetic acid and formic acid; and halogenated solvents such as dichloromethane and carbon tetrachloride; as well as mixtures thereof.

Once the mixture is formed, it is then heated to crosslink the polymeric material. Generally, the entire substrate or the entire coated portion of the substrate is heated to activate the coating, though a portion (e.g., less than 50%) of the substrate may be kept at a lower temperature than the remaining substrate if desired. The elevated temperature is generally maintained sufficiently long to at least partially activate the coating (e.g., from about 30 seconds to about 30 minutes). Heating is generally carried out at temperatures less than the melting point or decomposition point of the substrate. For example, in some embodiments, heating is carried out at a temperature of from about 100°C to about 300°C, in some embodiments from about 170°C to about 300°C, and in some embodiments, from about 170°C to about 250°C. The use of such low curing temperatures allows, in some embodiments, the resulting substrate to have an activated carbon coating without substantially sacrificing the flexibility or other mechanical properties of the substrate. Further, such low curing temperatures also allow for the use of polymers having low softening or decomposition temperatures (e.g., polyester) that are commonly employed in absorbent articles. In addition, not only does heating crosslink and activate the polymeric material, it also forms a durable coating that will generally remain present on the substrate during use. In one embodiment, the activated carbon coating on the substrate does not rub off to a significant degree when the coating is rubbed between the fingers of the human hand.

During activation, an integral coating of activated carbon can be formed around fibers or other components of the substrate, as opposed to the discretely attached particles that would result by adhesively attaching activated carbon particles to the substrate. Without wishing to be bound by theory, the activated carbon coating formed according to the present invention can be durably held in place on the substrate by either formation of a network that surrounds the substrate material and prevents release of the activated carbon, or by chemical bonds (covalent bonds, van der Waal bonds, etc.) between the activated carbon and the substrate material, or both. The coating can be substantially homogenous in chemical composition or in the mass distribution of activated carbon around the substrate material (e.g., a substantially uniform coating of integral activated carbon as opposed to discreet particles attached with an adhesive).

If desired, activation may take place in one or more incremental steps over a succession of temperatures to increase the concentration of porosity in the coating and minimize the amount of coating that is volatilized. Optionally, the cured coating may be further activated to produce a higher surface area by further heating in the presence of an inert gas or air. Selection of the specific polymeric material, chemical activation agent and its concentration, along with the activation temperature and time, will determine the specific surface area, pore size distribution and surface chemistry of the activated carbon coating. For example, low activation temperatures can be used to produce high surface area activated carbon coating fibers.

The characteristics of the resulting activated carbon coating generally vary based on the amount and type of the polymeric material and activation agent utilized. For example, in some embodiments, the amount of carbon in the coating is less than about 85 wt%, in some embodiments less than about 80 wt%, in some embodiments, from about 50 wt% to about 80 wt%, and in some embodiments, from about 60 wt.% to about 75 wt% of the substrate. In addition, the yield of activated carbon in the coating (the weight of activated carbon coating divided by the weight of coating mixture) may be at least about 50%, in some embodiments at least about 60%, in some embodiments at least about 80%, and in some embodiments, at least about 90%. Further, the resulting coating may have a B.E.T. surface area (measured using a "Quantachrome Autsorb-1" available from Quantachrome Instruments, Inc. of Boynton Beach, Florida) of at least about 50 m$^2$/g and an average pore size of from about 5 Angstroms (A) to about 35 A. Prior to heating, the coating mixture may have a surface area of up to about 10 m$^2$/g.

The solutions to be coated onto a substrate can have a viscosity of at least about 1 centipoise (cp), in some embodiments at least about 5 cp, in some embodiments at least about 10 cp, and in some embodiments, at least about 50 cp. If desired, thickeners and/or surfactants can be used to apply the coating material to the polymeric substrate. In one embodiment, the coating can be prepared as a foam that can collapse during heat treatment to increase the basis weight of the applied coating. Foams can be prepared by agitation of the solution in the presence of a surfactant. Thickeners, such as sodium alginate, xanthan gum, gum arabic, guar gum, sodium alginate, polyvinyl alcohol, bentonite, laponite, kaolin, and the like, may be used in the present invention.

## B. Substrates

Any of a variety of different substrates may be incorporated with the activated carbon coating in accordance with the present invention. For instance, nonwoven fabrics, woven fabrics, knit fabrics, wet-strength paper, films, foams, etc., may be applied with an activated carbon coating. When utilized, the nonwoven fabrics may include, but are not limited to, spunbonded webs (apertured or non-apertured), meltblown webs, bonded carded webs, air-laid webs, coform webs, hydraulically entangled webs, and the like. Generally, some or all of the fibers used to form the nonwoven fabric have a softening temperature that is higher than the temperatures needed to form the activated carbon coating. One or more components of such fibers may have, for instance, a softening temperature of from about 100°C to about 400°C, in some embodiments from about 100°C to about 300°C, and in some embodiments, from about 150°C to about 250°C. Examples of such fibers may include, but are not limited to, synthetic fibers (e.g., polyethylene, polypropylene, polyethylene terephthalate, nylon 6, nylon 66, KEVLAR™, syndiotactic polystyrene, liquid crystalline polyesters, etc.); cellulosic fibers (softwood pulp, hardwood pulp, thermomechanical pulp, etc.); combinations thereof; and the like. The substrate may also be characterized in terms of a degradation temperature, i.e., the temperature at which the uncoated fabric loses at least 50% of its tensile strength relative to an unheated fabric when the fabric is heated to that temperature for one hour in a normal atmosphere of air, then air cooled to room temperature over a period of two hours and then tested for tensile strength using a 7.62 cm (3-inch) wide sample with a gage length of 7.62 cm (3 inches) and a crosshead speed of 25.4 cm (10 inches) per minute. The degradation temperature can be less than about 450°C, in some embodiments less than about 250°C, and in some embodiments, less than about 200°C.

To further enhance the flexibility of the substrate, it contains a stretchable component that, upon application of a force, is stretchable to a stretched, biased length which is at least about 120%, and in some embodiments, at least about 150% its unstretched length. Optionally, the stretchable material will also recover at least about 50% of its elongation upon release of the stretching, biasing force. In some instances, an elastomeric material can enhance the flexibility of the substrate by enabling it to be more easily bent and distorted. When present in a substrate, the elastomeric material takes on various forms. For example, the elastomeric material can make up the entire substrate or form a portion of the substrate. In some embodiments, for instance, the elastomeric material can contain elastic strands or sections uniformly or randomly distributed throughout the substrate. Alternatively, the elastomeric component can be an elastic film or an elastic nonwoven web, such as an apertured web of elastomeric material comprising at least about 25% open area. The elastomeric component may be a single layer or a multi-layered material.

Although any elastomeric material may generally be used, it is often desired to select an elastomeric material that has a softening temperature greater than the activation temperature for the carbon coating. For instance, some suitable "high softening temperature" elastomeric materials that can be used, include, but are not limited to, fluoropolymers such as Viton® polymers sold by DuPont that can withstand temperatures of up to about 200°C; Kalrez® perfluoropolymers sold by DuPont that can withstand temperatures of up to about 300°C; highly saturated nitrile polymers; silicone polymers; ethylene vinyl acetate polymers, polyacrylate elastomers; and other elastomers and flexible polymers known in the art. Still other suitable elastomeric materials that may be used in the present invention include diblock, triblock, or multi-block elastomeric copolymers, such as olefinic copolymers (e.g., styrene-isoprenestyrene, styrene-butadiene-styrene, styrene-ethylene/butylene-styrene, or styreneethylene/propylene-styrene); polyurethanes; polyamides; polyesters; and the like. Other examples of suitable elastomeric materials are described in U.S. Patent No. 6,362,389 to McDowall, et al.

When incorporating an elastomeric component containing an elastomeric material, such as described above, into a substrate, it is sometimes desired that the elastomeric component include an elastic laminate that contains an elastomeric material with one or more other layers, such as foams, films, apertured films, and/or nonwoven webs. An elastic laminate generally contains layers that can be bonded together so that at least one of the layers has the characteristics of an elastic polymer. The elastic material used in the elastic laminates can be made from materials, such as described above, that are formed into films, such as a microporous film, fibrous webs, such as a web made from meltblown fibers, spunbond fibers, foams, and the like.

For example, in one embodiment, the elastic laminate can be a "neck-bonded" laminate. A "neck-bonded" laminate refers to a composite material having at least two layers in which one layer is a necked, non-elastic layer and the other layer is an elastic layer. The resulting laminate is thereby a material that is elastic in the cross-direction. Some examples of neck-bonded laminates are described in U.S. Patent Nos. 5,226,992, 4,981,747, 4,965,122, and 5,336,545, all to Morman. The elastic laminate can also be a "stretch-bonded" laminate, which refers to a composite material having at least two layers in which one layer is a gatherable layer and in which the other layer is an elastic layer. The layers are joined together when the elastic layer is in an extended condition so that upon relaxing the layers, the gatherable layer is gathered. For example, one elastic member can be bonded to another member while the elastic member is extended at least about 25 percent of its relaxed length. Such a multilayer composite elastic material may be stretched until the nonelastic layer is fully extended.

For example, one suitable type of stretch-bonded laminate is a spunbonded laminate, such as disclosed in U.S. Patent No. 4,720,415 to VanderWielen et al. Another suitable type of stretch-bonded laminate is a continuous filament spunbonded laminate, such as disclosed in U.S. Patent No. 5,385,775 to Wright. For instance, Wright discloses a composite elastic material that includes: (1) an anisotropic elastic fibrous web having at least one layer of elastomeric meltblown fibers and at least one layer of elastomeric filaments autogenously bonded to at least a portion of the elastomeric meltblown fibers, and (2) at least one gatherable layer joined at spaced-apart locations to the anisotropic elastic fibrous web so that the gatherable layer is gathered between the spaced-apart locations. The gatherable layer is joined to the elastic fibrous web when the elastic web is in a stretched condition so that when the elastic web relaxes, the gatherable layer gathers between the spaced-apart bonding locations. Other composite elastic materials are described and disclosed in U.S. Patent Nos. 4,789,699 to Kieffer et al., 4,781,966 to Taylor, 4,657,802 to Morman, and 4,655,760 to Morman et al.

In one embodiment, the elastic laminate can also be a necked stretch bonded laminate. As used herein, a necked stretch bonded laminate is defined as a laminate made from the combination of a neck-bonded laminate and a stretch-bonded laminate. Examples of necked stretch bonded laminates are disclosed in U.S. Patent Nos. 5,114,781 and 5,116,662. Of particular advantage, a necked stretch bonded laminate can be stretchable in both the machine and cross-machine directions. Creped nonwoven materials can also be used. Exemplary creped nonwoven webs are described in U.S. Patent Nos. 4,810,556 to Kobayashi, et al.; 6,197,404 to Varona; and 6,150,002 to Varona.

Other stretchable materials may also be used as the substrate. For example, stretchable polymeric meshes, such as polyester and nylon meshes and combinations thereof, may be used in the present invention. Examples of some suitable polyester and nylon meshes include, but are not limited to, 0.8 mm Polyester Mosquito Netting (Product FMN008), 1.5 mm Polyester Hex-Mesh (Product FMN001), 3 mm Nylon Hex-Mesh (Product FMN003), 840 x 1680 Denier Nylon Leno Mesh (Product FLM168), 6 mm Nylon Hex-Mesh (Product FMN006), and Nylon Spectra/Mesh™, all of which are available from American Home & Habitat (King George, Virginia). Suitable meshes can have substantial open area in the relaxed state (while not actively being stretched), such as about 25% open area or greater, about 50% open area or greater, or about 80% open area or greater. Scrim materials are one form of mesh that can be considered.

The permeability of the substrate may be varied for a particular application. For example, in some embodiments, the substrates may contain a material having an average pore size that renders it permeable to liquids. Liquid permeability may enhance the absorption characteristics of the substrate, and also render it more flexible. For instance, relatively large pores do not tend to become as blocked by activated carbon as smaller pores. As a result, the open pores provide the substrate room to extend without constraint by activated carbon, which is relatively stiff. In this manner, the pore size can facilitate the flexibility of the substrate. Examples of average pore sizes that can improve substrate flexibility are those in the range of 0.1 to about 1000 micrometers, in some embodiments from about 0.1 to about 10 milllimeters, and in some embodiments, from about 0.3 to about 5 millimeters.

When formed from a liquid-permeable substrate, it is typically desired that the substrate, after being coated with activated carbon, remain relatively porous so that it is absorbent when incorporated into an absorbent article. The porosity may be maintained in a variety of ways. For instance, the resulting substrate may be apertured using known techniques. Moreover, the activated carbon coating can be applied such a manner that the particles do not substantially block the pores of the substrate. Regardless of the technique utilized to maintain porosity, the activated carbon coated substrate may have sufficient porosity such that about 0.566 $m^3$ (20 cubic feet) of air or greater can flow through 0.092 $m^2$ (1 square foot) of the substrate in 1 minute under an air pressure differential of 125 Pascals 1.27 cm (0.5 inches) of water). In other words, such a substrate is said to have an air permeability of about 0.566 $m^3$ (20 cubic feet) per minute (cfm) or greater. Air permeability (volumetric air flow per 0.0929 $m^2$ (square foot) of material under an air pressure differential of 125 Pascals) may be measured in a variety of ways. For example, "Frazier Air Permeability" is determined according to Federal Test Standard 191A, Method 5450 with a Frazier Air Permeability Tester (Frazier Precision Instrument Co., Gaithersburg, Md.), and is reported as an average of 3 sample readings. It should be understood, however, that other techniques may also be used to determine porosity. For instance, an alternative technique is described below in Example 13, which is believed to give essentially the same results as Frazier Air Permeability. In general, the air permeability of a fabric formed according to the present invention may range from about 20 cfm to about 500 cfm, in some embodiments from about 50 cfm to about 400 cfm, and in some embodiments, from about 75 cfm to about 300 cfm, under an air pressure differential of 125 Pascals.

Other materials that may be treated according to the present invention include materials made from one or more of the following polymers: liquid crystal polymers, such as Vectra™; Celanex® or Vandar® thermoplastic polyester; Riteflex® thermoplastic polyester elastomer; long fiber reinforced thermoplastics such as Compel®, Celstran®, and Fiberod® products; Topas® cyclic-olefin copolymer; Duracon®, Celcon®, and Hostaform® acetal copolymers; Fortron® polyphenylene sulfide; and Duranex™ thermoplastic polyester (PBT), all of which are available from Ticona Corp. (Summit, New Jersey).

The substrate may be applied with various treatments to impart desirable characteristics. For example, the substrate may be treated with liquid-repellency additives, antistatic agents, surfactants, colorants, antifogging agents, fluorochemical blood or alcohol repellents, lubricants, and/or antimicrobial agents. In addition, the substrate may also be subjected to an electret treatment. The electret treatment imparts an electrostatic charge to the substrate to improve its filtration efficiency. The charge may include layers of positive or negative charges trapped at or near the surface of the polymer, or charge clouds stored in the bulk of the polymer. The charge may also include polarization charges that are frozen in alignment of the dipoles of the molecules. Techniques for subjecting the substrate to an electret treatment are well known by those skilled in the art. Examples of such techniques include, but are not limited to, thermal, liquid-contact, electron beam and corona discharge techniques. In one particular embodiment, the electret treatment is a corona discharge technique, which involves subjecting the substrate to a pair of electrical fields that have opposite polarities. Other methods for forming an electret material are described in U.S. Patent Nos. 4,215,682 to Kubik. et al.;

4,375,718 to Wadsworth; 4,592,815 to Nakao; 4,874,659 to Ando; 5,401,446 to Tsai, et al.; 5,883,026 to Reader, et al. ; 5,908,598 to Rousseau, et al.; 6,365,088 to Knight, et al.

C. Application of the Activated Carbon Coating

A variety of techniques may be utilized to apply the coating of the polymeric material and the activation agent to the substrate. For instance, in one embodiment, a polymeric material is initially dissolved in a solvent, mixed with a chemical activation agent, and then applied to the substrate material. Alternatively, the chemical activation agent may initially be applied to the substrate material. Thereafter, the polymeric material may be applied to the substrate material. Moreover, the polymeric material may also initially be applied to the substrate material prior to application of the chemical activation agent.

When the polymeric material and/or chemical activation agent are applied to a formed substrate, for instance, any known method of application may be utilized, such as print, spraying, contact coated, blade, saturant, coating, droplet throw, paint, and foam applications. For example, in one embodiment, the polymeric material, the chemical activation agent, or a mixture thereof can be saturated into the substrate. Moreover, in another embodiment, the polymeric material, the chemical activation agent, or a mixture thereof can be printed onto at least one side of the substrate, and, in some cases to both outer surfaces of the substrate.

The add-on level of the activated carbon coating to the substrate may generally be varied as desired. The "add-on level" refers to the mass of the activated carbon coating divided by the oven-dry mass of the uncoated substrate, multiplied by 100%. For example, a 5-gram non-woven web with 5 grams of added activated carbon would have an add-on of 100%. The add-on level can be expressed in terms of total activated carbon relative to total substrate weight, or, in the case of heterogeneously treated substrates, the "local" add-on value can be expressed in terms of the mass of the activated carbon in a particular region coated with activated carbon relative to the mass of the fraction of the substrate that for which at least one surface has been provided with the activated carbon coating. Generally speaking, a lower add-on level results in a lower increase in substrate stiffness, while a higher add-on level results in the presence of a greater amount of activated carbon on the substrate. Thus, in some embodiments, the activated carbon can have an add-on level of from about 1 % to about 300% of the mass of the substrate, in some embodiments from about 5% to about 200% of the mass of the substrate, in some embodiments from about 5% to about 100% of the mass of the substrate, and in some embodiments, from about 5% to about 50% of the mass of the substrate.

The resulting activated carbon substrate is capable of performing multiple functions when incorporated into an absorbent article. For example, an absorbent substrate may continue to function in its absorbent capacity within the article, but also have additional functions stemming from the presence of activated carbon therein, such as adsorbing odor-producing materials.

The surface chemistry of the activated carbon coating may be tailored to optimize odor reduction or other additional functions performed by the substrate. For example, basic groups are desired on the activated carbon substrate for adsorbing acidic compounds, such as isovaleric acid or hydrochloric acid. Basic groups can be introduced by treatment with ammonia at elevated temperatures or by other treatments known in the art. In one embodiment, to form a basic surface chemistry, nitrogen containing polymeric materials may be used, such as polyacrylonitrile (PAN), with an activation agent (e.g., zinc chloride). In one particular embodiment, this coating mixture is heated to about 300°C to about 400°C for about 2 minutes to about 24 hours. The resulting assemblies have B.E.T. surface areas of about 400 and 1200 $m^2$/g and a nitrogen content ranging from about 12 wt.% to about 20 wt.% based upon the weight percent of activated carbon coating. Optionally, much higher temperatures, e.g., up to about 900°C, may be used for increased surface areas.

In addition, acidic groups are desired on the activated carbon substrate for adsorbing basic compounds, such as those having ammonium moieties. Acidic groups can be introduced by treating the fibers at elevated temperatures in the presence of steam, carbon dioxide, nitric acid, and the like. In one embodiment, oxygen containing polymeric materials, such as polyvinyl alcohol (PVA) or cellulose, may be used with an activation agent (e.g., phosphoric acid). In one particular embodiment, such a coating mixture is heated to from about 150°C to about 300°C for about 2 minutes to about 24 hours.

To maintain absorbency, flexibility, or some other characteristic of the substrate, it may sometimes be desired to apply the polymeric material, the chemical activation agent, or a mixture thereof so as to cover less than about 100% of the surface area of the substrate, in some embodiments from about 10% to about 80% of the surface area of the substrate, and in some embodiments, from about 20% to about 60% of the surface area of each side of the substrate. For instance, in one particular embodiment, the polymeric material, the chemical activation agent, or a mixture thereof is applied to the substrate in a preselected pattern (e.g., reticular pattern, diamond-shaped grid, dots, and the like). Although not required, such a patterned coating may provide sufficient activation to the substrate without covering a substantial portion of the surface area of the substrate. This may be desirable to optimize flexibility, absorbency, or other characteristics of the resulting absorbent article.

In addition, a patterned coating may also provide different functionality to each zone. For example, in one embodiment, the substrate is treated with two or more patterns of activated carbon regions that may or may not overlap. The regions may be on the same or different surfaces of the substrate. For example, in one embodiment, one surface of a substrate is treated with a polyacrylonitrile resin while another surface is treated with a polyvinyl alcohol resin. Each surface is then activated, such as described above, so that the resulting substrate has surfaces with different activated carbon coatings. This may allow, for instance, one surface to adsorb a basic odor-producing material, such as ammonia and/or triethylamine, while the other surface may adsorb an acidic odor-producing material, such as isovaleric acid. Other treatment conditions may also be varied to achieve different degrees of carbon activation. For instance, in one embodiment, a single polymeric material is applied to the substrate. However, during chemical activation, one portion of the substrate is exposed to a first activating gas, while another portion of the substrate is exposed to a second activating gas. Moreover, additives may be used in the activated carbon coatings to vary the pore size and/or layer thickness of different coatings. The use of additives may also result in varied functional groups within each activated carbon coating. For instance, some suitable additives include, but are not limited to, metal compounds, organometallic compounds, pigments, mineral fillers, catalysts, acids or bases, and the like.

Besides having functional benefits, the activated carbon substrates may also have various aesthetic benefits as well. For example, the substrate may be incorporated with the activated carbon coating without having the black color commonly associated with activated carbon. For example, in one embodiment, a relatively thin layer of the activated carbon coating is applied to a white or light-colored substrate so that the resulting substrate has a grayish or bluish color. In another embodiment, activation of the coating is halted prior to completion to leave a coating having a color other than black. In addition, the substrate may also be applied with patterned regions of the activated carbon coating to form a substrate having differently colored regions.

D. Absorbent Articles

In some embodiments, the flexible substrate may be incorporated into an absorbent article where it can function to reduce odor. For example, the activated carbon substrate may be incorporated into regions of the absorbent article that are likely to remain relatively dry during use, such as the wings of a sanitary napkin, waistbands or leg cuffs of a diaper, or the remote ends or sides of an article positioned a certain distance from the zone configured to receive bodily fluids. By being positioned in a relatively dry area of the absorbent article, the activated carbon substrate can reduce the odor of the article without having its odor-reducing capabilities substantially weakened by wetting. The activated carbon substrate may form the entire absorbent article, or may form only a portion of the article. For example, in some embodiments, the activated carbon substrate may constitute a surge layer, cover layer, transfer delay layer, etc. of an absorbent article, such as a sanitary napkin or diaper. When utilized in this manner, the substrate may still function in the manner desired, but also be capable of reducing odor. For instance, cover layers of sanitary napkins are configured to quickly absorb fluids and wick them towards the inner layers of the napkin. When utilized as a cover layer, for instance, the activated carbon substrate may still function to absorb fluids.

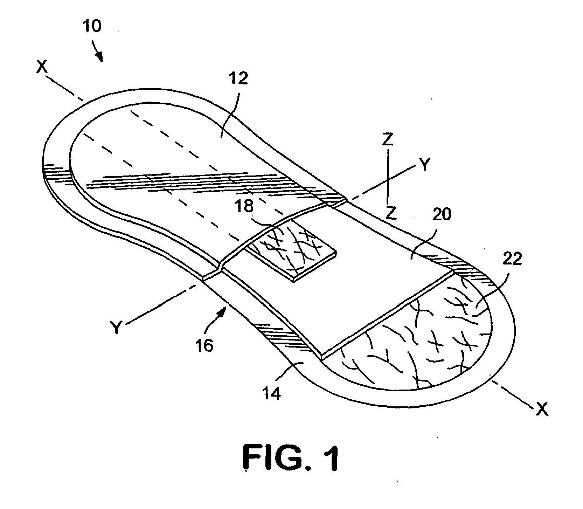

In this regard, various embodiments of an absorbent article that may be formed according to the present invention will now be described in more detail. For purposes of illustration only, an absorbent article 10 is shown in Fig. 1 as a sanitary napkin for feminine hygiene. However, as discussed above, the invention may be embodied in other types of absorbent articles, such as diapers, diaper pants, feminine napkins, children's training pants, and the like. Nonetheless, in the illustrated embodiment, the absorbent article 10 includes a cover 12, a baffle 14, and an absorbent core 16, any of which may contain the activated carbon substrate of the present invention. The absorbent core 16 is positioned inward from the outer periphery of the absorbent article 10 and includes a body-facing surface positioned adjacent the cover 12 and a garment-facing surface positioned adjacent the baffle 14.

The cover 12 is generally designed to contact the body of the user and is liquid-permeable. The cover 12 can surround the absorbent core 16 so that it completely encases the absorbent article 10. Alternatively, the cover 12 and the baffle 14 can extend beyond the absorbent core 16 and be peripherally joined together, either entirely or partially, using known techniques. Typically, the cover 12 and the baffle 14 are joined by adhesive bonding, ultrasonic bonding, or any other suitable joining method known in the art.

The liquid-permeable cover 12 is sanitary, clean in appearance, and somewhat opaque to hide bodily discharges collected in and absorbed by the absorbent core 16. The cover 12 further exhibits good strike-through and rewet characteristics permitting bodily discharges to rapidly penetrate through the cover 12 to the absorbent core 16, but not allow the body fluid to flow back through the cover 12 to the skin of the wearer. For example, some suitable materials that can be used for the cover 12 include nonwoven materials, perforated thermoplastic films, or combinations thereof. A nonwoven fabric made from polyester, polyethylene, polypropylene, bicomponent, nylon, rayon, or like fibers may be utilized. For instance, a white uniform spunbond material is particularly desirable because the color exhibits good masking properties to hide menses that has passed through it. U.S. Patent Nos. 4,801,494 to Datta, et al. and 4,908 026 to Sukiennik. et al. teach various other cover materials that can be used in the present invention. If desired, the cover 12 may be incorporated with an activated carbon coating in accordance with the present invention to enable it to better function in reducing odors of bodily fluids.

The cover 12 can also contain a plurality of apertures (not shown) formed therethrough to permit body fluid to pass more readily into the absorbent core 16. The apertures can be randomly or uniformly arranged throughout the cover 12, or they can be located only in the narrow longitudinal band or strip arranged along the longitudinal axis X--X of the absorbent article 10. The apertures permit rapid penetration of body fluid down into the absorbent core 16. The size, shape, diameter any number of apertures can be varied to suit one's particular needs.

As stated above, the absorbent article also includes a baffle 14. The baffle 14 is generally liquid-impermeable and designed to face the inner surface, i.e., the crotch portion of an undergarment (not shown). The baffle 14 can permit a passage of air or vapor out of the absorbent article 10, while still blocking the passage of liquids. Any liquid-impermeable material can generally be utilized to form the baffle 14. For example, one suitable material that can be utilized is a microembossed polymeric film, such as polyethylene or polypropylene. In particular embodiments, a polyethylene film is utilized that has a thickness in the range of about 0.2 mils to about 5.0 mils, and particularly between about 0.5 to about 3.0 mils. If desired, the baffle 14 may be incorporated with an activated carbon coating in accordance with the present invention to enable it to better function in reducing odors of bodily fluids.

As indicated above, the absorbent article 10 also contains an absorbent core 16 positioned between the cover 12 and the baffle 14. In the illustrated embodiment, for example, the absorbent core 16 contains three separate and distinct absorbent members 18, 20 and 22, any of which may contain the activated carbon substrate of the present invention. It should be understood, however, that any number of absorbent members can be utilized in the present invention. For example, in one embodiment, only the absorbent member 22 may be utilized.

As shown, the first absorbent member 18, or intake member, is positioned between the cover 12 and the second absorbent member 20, or transfer delay member. The intake member 18 represents a significant absorbing portion of the absorbent article 10 and has the capability of absorbing at least about 80%, particularly about 90%, and more particularly about 95% of the body fluid deposited onto the absorbent article 10. In terms of amount of body fluid, the intake member 18 can absorb at least about 20 grams, particularly about 25 grams, and more particularly, about 30 or more grams of body fluid.

The intake member 18 can generally have any shape and/or size desired. For example, in one embodiment, the intake member 18 has a rectangular shape, with a length equal to or less than the overall length of the absorbent article 10, and a width less than the width of the absorbent article 10. For example, a length of between about 150 mm to about 300 mm and a width of between about 10 mm to about 40 mm can be utilized.

Typically, the intake member 18 is made of a material that is capable of rapidly transferring, in the z-direction, body fluid that is delivered to the cover 12. Because the intake member 18 is generally of a dimension narrower than the absorbent article 10, the sides of the intake member 18 are spaced away from the longitudinal sides of the absorbent article 10 and the body fluid is restricted to the area within the periphery of the intake member 18 before it passes down and is absorbed into the transfer delay member 20. This design enables the body fluid to be combined in the central area of the absorbent article 10 and to be wicked downward.

In general, any of a variety of different materials are capable of being used for the intake member 18 to accomplish the above-mentioned functions. For example, airlaid cellulosic tissues may be suitable for use in the intake member 18. The airlaid cellulosic tissue can have a basis weight ranging from about 10 grams per square meter (gsm) to about 300 gsm, and in some embodiments, between about 100 gsm to about 250 gsm. In one embodiment, the airlaid cellulosic tissue has a basis weight of about 200 gsm. The airlaid tissue can be formed from hardwood and/or softwood fibers. The airlaid tissue has a fine pore structure and provides an excellent wicking capacity, especially for menses.

A second absorbent member 20, or transfer delay member, is also positioned vertically below the intake member 18. In some embodiments, the transfer delay member 20 contains a material that is less hydrophilic than the other absorbent members, and may generally be characterized as being substantially hydrophobic. For example, the transfer delay member 20 may be a nonwoven fibrous web composed of a relatively hydrophobic material, such as polypropylene, polyethylene, polyester or the like, and also may be composed of a blend of such materials. One example of a material suitable for the transfer delay member 20 is a spunbond web composed of polypropylene, multi-lobal fibers. Further examples of suitable transfer delay member materials include spunbond webs composed of polypropylene fibers, which may be round, tri-lobal or poly-lobal in cross-sectional shape and which may be hollow or solid in structure. Typically the webs are bonded, such as by thermal bonding, over about 3% to about 30% of the web area. Other examples of suitable materials that may be used for the transfer delay member 20 are described in U.S. Patent Nos. 4,798,603 to Meyer, et al. and 5,248,309 to Serbiak, et al. To adjust the performance of the invention, the transfer delay member 20 may also be treated with a selected amount of surfactant to increase its initial wettability.

The transfer delay member 20 can generally have any size, such as a length of about 150 mm to about 300 mm. Typically, the length of the transfer delay member 20 is approximately equal to the length of the absorbent article 10. The transfer delay member 20 can also be equal in width to the intake member 18, but is typically wider. For example, the width of the transfer delay member 20 can be from between about 50 mm to about 75 mm, and particularly about 48 mm.

The transfer delay member 20 of the absorbent core 16 typically has a basis weight less than that of the other absorbent members. For example, the basis weight of the transfer delay member 20 is typically less than about 150 grams per square meter (gsm), and in some embodiments, between about 10 gsm to about 100 gsm. In one particular embodiment, the transfer delay member 20 is formed from a spunbonded web having a basis weight of about 30 gsm.

Besides the above-mentioned members, the absorbent core 16 also includes a composite member 22. For example, the composite member 22 can be a coform material. In this instance, fluids can be wicked from the transfer delay member 20 into the absorbent member 22. The composite absorbent member 22 may be formed separately from the intake member 18 and/or transfer delay member 20, or can be formed simultaneously therewith. In one embodiment, for example, the composite absorbent member 22 can be formed on the transfer delay member 20 or intake member 18, which acts a carrier during the coform process described above.

The absorbent article 10 may also contain other components as well. For instance, in some embodiments, the lower surface of the baffle 14 can contain an adhesive for securing the absorbent article 10 to an undergarment. In such instances, a backing (not shown) may be utilized to protect the adhesive side of the absorbent article 10 so that the adhesive remains clean prior to attachment to undergarment. The backing can generally have any desired shape or dimension. For instance, the backing can have a rectangular shape with dimension about 17 to about 21 cm in length and about 6.5 to 10.5 cm in width. The backing is designed to serve as a releasable peel strip to be removed by the user prior to attachment of the absorbent article 10 to the undergarment. The backing serving as a releasable peel strip can be a white Kraft paper that is coated on one side so that it can be released readily from the adhesive side of the absorbent article 10. The coating can be a silicone coating, such as a silicone polymer commercially available from Akrosil of Menasha, Wisconsin.

Once formed, the absorbent article 10 generally functions to absorb and retain fluids, such as menses, blood, urine, and other excrements discharged by the body during a menstrual period. For example, the intake member 18 can allow the body fluid to be wicked downward in the z-direction and away from the cover 12 so that the cover 12 retains a dry and comfortable feel to the user. Moreover, the intake member 18 can also absorb a significant amount of the fluid. The transfer delay member 20 initially accepts fluid from the intake member 18 and then wicks the fluid along its length and width (-x and -y axis) before releasing the fluid to the composite absorbent member 22. The composite absorbent member 22 then wicks the fluid along its length and width (-x and -y axis) utilizing a greater extent of the absorbent capacity than the transfer delay member 20. Therefore, the composite absorbent member 22 can become completely saturated before the fluid is taken up by the transfer delay member 20. The fluid is also wicked along the length of the transfer delay member 20 and the composite absorbent member 22, thereby keeping the fluid away from the edges of the absorbent article 10. This allows for a greater utilization of the absorbent core 16 and helps reduce the likelihood of side leakage.

Although one embodiment of an absorbent article has been described above that may utilize the activated carbon substrate of the present invention, it should be understood that other absorbent article configurations are also included within the scope of the present invention. For instance, other absorbent configurations are described in U.S. Patent Nos. 5,197,959 to Buell; 5,085,654 to Buell; 5,634,916 to Lavon, et al.; 5,569,234 to Buell, et al.; 5,716,349 to Taylor, et al.; 4,950,264 to Osborn; 5,009,653 to Osborn; 5,509,914 to Osborn; 5,649,916 to DiPalma, et al.; 5,267,992 to Van Tillburg; 4,687,478 to Van Tillburg; 4,285,343 to McNair; 4,608,047 to Mattingly; 5,342,342 to Kitaoka; 5,190,563 to Herron, et al.; 5,702,378 to Widlund. et al.; 5,308,346 to Sneller, et al.; 6,110,158 to Kielpikowski; and WO 99/00093 to Patterson, et al. For instance, in one embodiment, the activated carbon substrate of the present invention is used to form the leg cuff of a diaper.

The present invention may be better understood with reference to the following examples.

### EXAMPLES 1-17

Examples 1-17 were prepared using the following protocols for the treatments specified:

### (A) Phenolic resin and ZnCl$_2$ coating

1. Add 15 grams of phenolic resin and 25-30 grams ZnCl$_2$ into 100 mL ethanol solvent, stir at room temperature until dissolved.
2. Dip coat the sample mat with the above solution (or use an airbrush).
3. Dry the coated sample in the air for 20 minutes.
4. Heat the sample in the oven at 100°C and 150°C. At each temperature, hold for 10 minutes.
5. Increase the temperature to 170°C and activate for 1 hour.
6. Soak the sample in 0.5 N HCl for 2 hours.

7. Wash the sample with deionized (DI) water several times.

8. Dry the sample in oven at 120°C for 0.5 hours.

## (B) Polyacrylonitrile (PAN) coating

1. Add 1.6 grams of PAN into 50 mL DMF (dimethylformamide) solvent and heat to 70-80°C until the PAN is completely dissolved. Then, let the solution cool down, add 3.2 grams of $ZnCl_2$, and continuously stir the solution until the $ZnCl_2$ is also dissolved. (Another method to make PAN solution: Add 32 mL DI water into 66 grams of $ZnCl_2$ and heat to 70-80°C. When dissolved, add 2 grams of PAN into the solution and heat to 70-80°C until dissolved again).

2. Dip coat samples with the above solution (or use an airbrush).

3. Quickly dip the coated sample into 2.5% $ZnCl_2$ + DI solution for a couple of seconds.

4. Dry the coated sample in the oven at 120°C for 30 minutes.

5. Activate the sample in the oven at 170°C for 0.5 hours.

6. Soak the sample in 0.5 N HCl for 2 hours.

7. Wash the sample with DI water several times.

8. Dry the sample in oven at 120°C for 0.5 hours.

## (C) Polyvinyl alcohol (PVA) coating

1. Add 1 gram of PVA into 10 mL DI water and heat to 80-90°C until dissolved. Then, let the solution cool down, add 1 mL $H_3PO_4$, and stir the solution for complete mixing.

2. Dip coat samples with the above solution (or use an airbrush).

3. Dry the coated sample in the oven at 120°C for 20 minutes.

4. Increase the temperature to 170°C and hold for 0.5 hours.

5. Wash the sample with 0.5 N NaOH several times to remove $H_3PO_4$.

6. Soak the sample in DI water for 2 hours, and then wash with DI water several times.

7. Dry the sample in oven at 120°C for 0.5 hours.

## (D) Cellulose in DMF

1. Add 10 grams of cellulose into 50 mL DMF and heat to 80-90°C until dissolved. Then, let the solution cool down, add 15 mL $H_3PO_4$, and stir the solution for complete mixing.

2. Dip coat samples with the above solution (or use an airbrush).

3. Dry and stabilize the sample at 140°C for 0.5 hours.

4. Activate the sample in the oven at 170°C for 1 hour.

5. Wash thoroughly with 0.5N NaOH to remove $H_3PO_4$.

6. Soak the sample in DI water for 2 hours, and then wash with DI water several times.

7. Dry the sample in oven at 120°C for 0.5 hours.

## (E) Cellulose in $ZnCl_2$ solution

1. Add 24 mL DI water into 66 grams of $ZnCl_2$, and heat to 70-80°C. When dissolved, add 10 grams of cellulose into the solution and heat to 70-80°C until dissolved again.

2. Dip coat samples with the above solution (or use an airbrush).

3. Dry and stabilize the sample at 140°C for 0.5 hours.

4. Activate the sample in the oven at 170°C for 1 hour.

5. Wash with 0.5N NaOH thoroughly to remove $H_3PO_4$.

6. Soak the sample in DI water for 2 hours, and then wash with DI water several times.

7. Dry the sample in oven at 120°C for 0.5 hours.

Generally speaking, forced air ovens were used at low temperature (approximately 110°C) to dry the samples and then a convection oven or tube furnace (for temperatures above 200°C) was used to activate the samples. The oven can be run under vacuum during activation, if desired.

## EXAMPLE 1

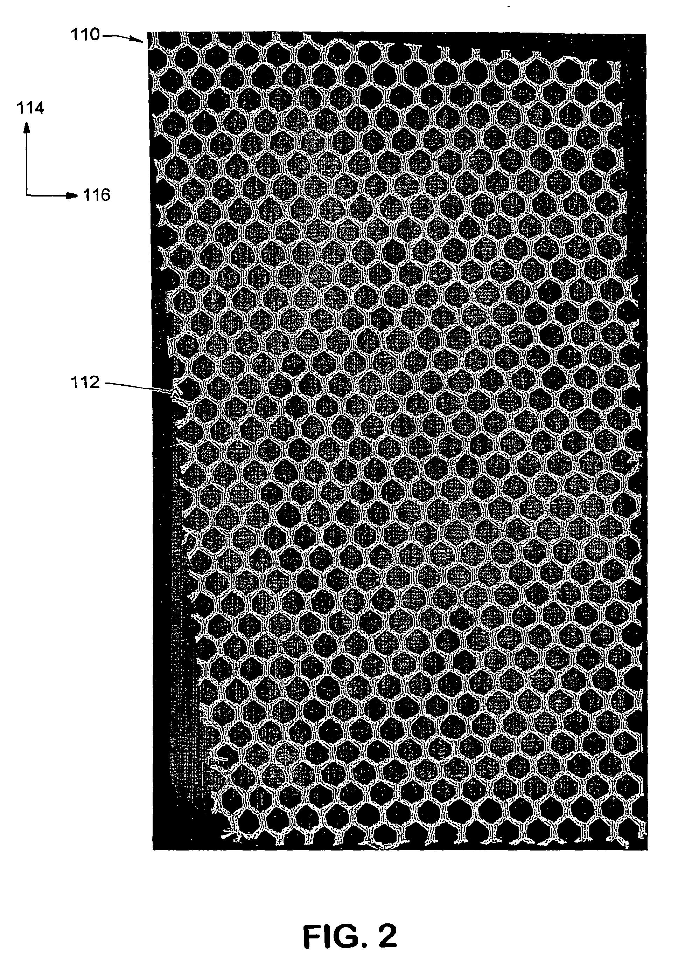

The ability to form an activated carbon coating on a substrate was demonstrated. The substrate was a polymeric mesh, hereafter known as "Nylon Mesh D" and was taken from a commercial wash towel sold under the name "Scrub & Rub" by Ostrow Textile, L.L.C. (Rock Hill, SC). Nylon Mesh D had a hexagonal pattern with openings approximately 2.8 millimeters wide and walls about 1 millimeter. Figure 2 shows a photograph of the mesh substrate 110 against a dark background. The melting point of the mesh was measured to be 252°C (within the normal range for nylon).

A 30.5 cm (12-inch) by 31.75 cm (12.5-inch) rectangle of the mesh had a mass of 4.3 grams, giving a basis weight of about 44 grams per square meter (gsm). The mesh had fibers 112 primarily oriented in a first direction (longitudinal direction) 114, orthogonal to the traverse direction 116. Tensile testing was conducted using an MTS Alliance RT/1 testing device operating with TestWorks 4 software on a PC computer under Windows 98. The sample was cut to 2.54 cm (1-inch) wide and 12.7 cm (5 inches) long (in the longitudinal direction of the mesh) and tested for tensile properties with a crosshead speed of 25.4. cm (10 inches) per minute and a gage length of 10.16 cm (4 inches).

In the longitudinal direction, the mean peak load at failure was 6515 grams of force (standard deviation was 881 grams of force, with 4 samples tested), with a peak stretch of 42.3% (std. dev. of 5.25% stretch). In the traverse direction, the mean peak load at failure was 4351 grams of force (standard deviation was 379 grams of force, with 5 samples tested), with a peak stretch of 60.4% (std. dev. of 7.73% stretch). Caliper measurement with an Enveco test device, operating with an applied load of 0.289 psi and a 5.64 cm (2.22-inch) diameter foot, was 0.44 millimeters.

The sample was then coated with PVA according to the PVA protocol above. In one run, it was activated at 170°C for 30 minutes, resulting in a flexible, stretchable, permeable activated carbon fabric having an add-on level of 12%, corresponding to 10.7 weight % activated carbon coating (i.e., 89.3% of the total mass of the treated activated carbon fabric is the substrate, and 10.7% is the added activated carbon-containing coating, so the add-on level is 10.7/89.3 *100% = 12%).

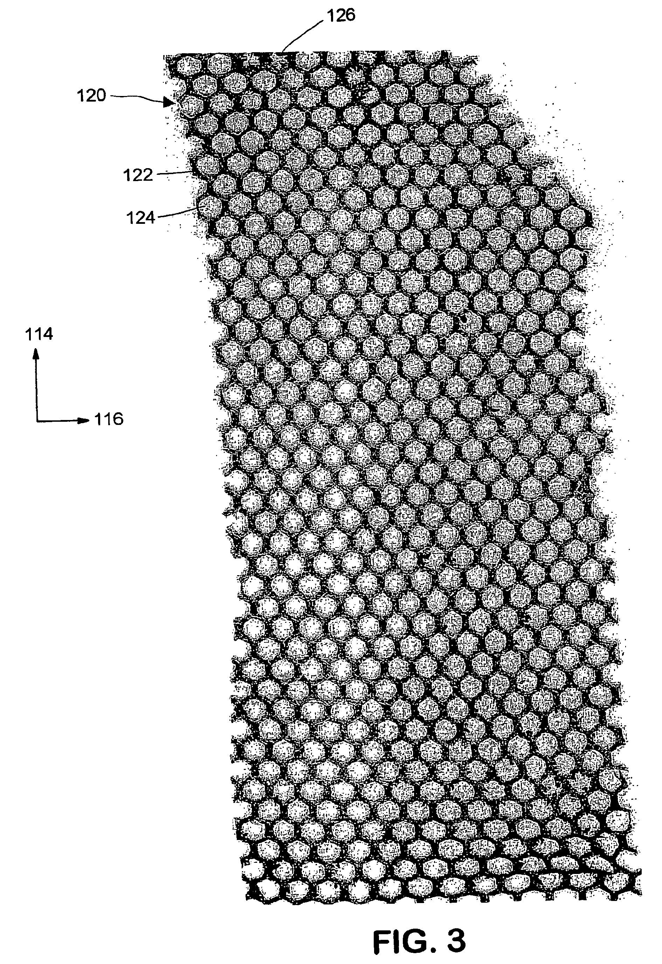

Figure 3 shows a photograph of the treated sample 120, which comprises a solid network 122 defining isolated open regions 124 through which fluid can pass. Some of the open regions 124 are partially occluded by flakes 126 attached to the solid network 122, which were apparently formed from the coating applied to the substrate. If desired, the flakes could be removed by further mechanical treatment, such as exposure to intense air flow from an air knife, brushing, flexing of the web as it bends around rollers, or other mechanical treatments.

Tensile properties of the sample 120 in Figure 3 were tested using a 1-inch by 4-inch strip cut from the slightly larger material shown, with the 4-inch direction in the longitudinal direction (the direction of highest tensile strength in the original fabric). Tensile testing was done with a crosshead speed of 10 inches per minute with the MTS Alliance RT/1 test device and a gage length of 3 inches. The measurement for the sample gave a tensile strength of 2953 grams of force (gf), with a peak stretch of 27.8%, and a mean TEA (Total Energy Absorbed) value of 121 gf * cm/cm$^2$. When the untreated sample was cut to the same dimensions and with the same orientation, and tested in the same manner (7.62 cm (3-inch) gage length, etc.), a single sample yielded a tensile strength (peak load) of 5023 gf and a stretch of 32.7%. The treated fabric appeared to have shrunk slightly (estimated 5-10%) relative to the original fabric.

On the basis of these tests, it appears that the activated carbon fabric retained about 60% of its initial tensile strength and about 90% of its initial stretch. However, regardless of the tensile properties of the individual fibers, very little force caused significant deformation of the web because of the very open network structure of the fabric.

In a second run, the mesh was activated at 225°C for 45 minutes. This treatment damaged the substrate.

In a third run, Nylon Mesh D was treated with PAN according to the protocol given above and activated at 225°C for 45 minutes, yielding an activated carbon fabric with 9.3% activated carbon. The fabric was relatively stiff.

In a fourth run, Nylon Mesh D was treated with cellulose in DMF and activated at 225°C for 45 minutes. The fabric was relatively stiff.

In a fifth run, Nylon Mesh D was dip coated with the PVA solution and activated under vacuum at 183°C, resulting in a pliable, stretchable mesh with 26.7 weight % activated carbon coating.

In a sixth run, another section of Nylon Mesh D was prepared as in the first run, being coated with PVA and activated at 170°C. Tensile testing with three samples (2.54 cm (1 inch) wide by 7.62 cm (3 inches) long) for one primary orientation of the mesh gave a mean peak load at failure of 5177 grams of force (st. dev. of 186.5 g), with a mean peak stretch of 43.1% (st. dev. of 1.40). The mean TEA (Total Energy Absorbed) was 318 grams of force * cm/cm$^2$. In the orthogonal direction, testing of a three samples gave a peak load of 3615 grams of force (st. dev. of 390 grams of force), with a mean peak stretch of 43.1% (st. dev. of 1.8) and a mean TEA of 302 grams of force * cm/cm$^2$.

## EXAMPLE 2

"Nylon Mesh A" was treated with activated carbon according to the present invention. Nylon Mesh A was taken from the cover material on a Custom Auto Care Squeegee Washer, product 17004 manufactured by Custom Accessories, Inc., of Niles, Illinois. This mesh had a finer structure than Nylon Mesh D of Example 1 and had a basis weight of about 47 grams per meters squared. The melting point was approximately 250°C. The mesh was coated with PVA according to the PVA protocol above. In one run, the coating was activated at 170°C for 30 minutes, resulting in a flexible, extensible, permeable activated carbon fabric having an add-on level of 12.1 % activated carbon. The melting point was approximately 250°C. The mesh was coated with PVA according to the PVA protocol above. In one run, the coating was activated at 170°C for 30 minutes, resulting in a flexible, extensible, permeable activated carbon fabric having 12.1-weight % activated carbon (13.6% add-on).

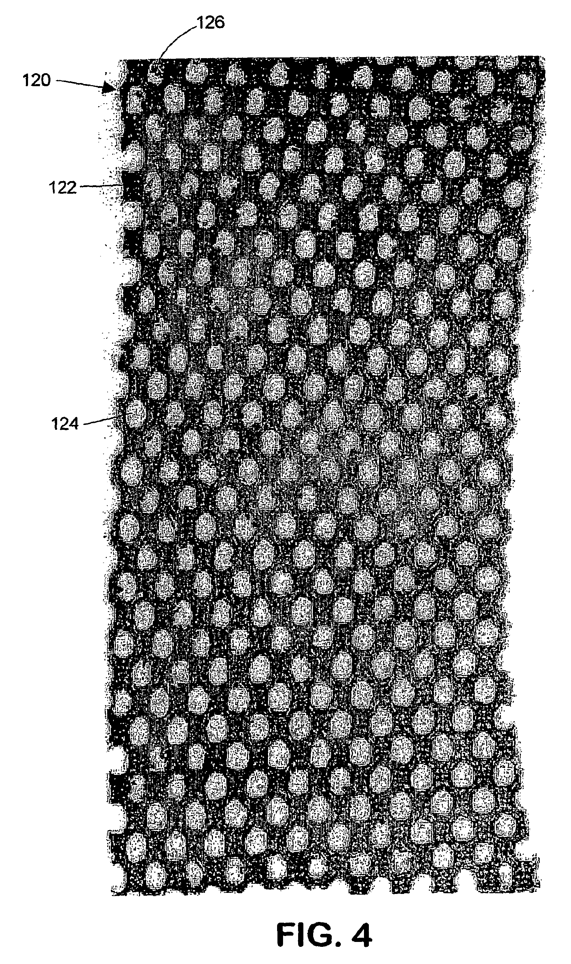

Figure 4 shows the resulting activated carbon fabric 120, which has dimensions of approximately 6.8 cm by 3.1 cm. From left to right, there were 9 openings with a 2.7 cm length, and approximately 29 staggered rows of holes from top to bottom. The openings were slightly greater than 2 millimeters wide and slightly less than 2 millimeters tall.

The fabric appeared to have good flexibility and durability. Rubbing the fabric between the fingers did not result in visible release of black particles.

Nylon Mesh A was also treated with PVA at 225°C for 45 minutes. This treatment caused the substrate to be relatively stiff.

## EXAMPLE 3

A sample was cut from a ScotchBrite™ abrasive scrubbing pad from 3M (St. Paul, Minnesota) and treated with PVA, as described above, at 225°C and 45 minutes. The resulting substrate had 16.3 weight % activated carbon solids (add-on of 19.5%) and was relatively coarse. Another run with another section of the pad gave an add-on level of 12.4%. Another sample was cut from the ScotchBrite™ pad was cut and treated with phenolic resin, as described above, at 225°C and 45 minutes. The resulting substrate had an add-on level of 35% and was relatively coarse.

## EXAMPLE 4

Several tests were conducted on a PGI 5928 fabric available from Polymer Group, Inc. (Mooresville, NC). PGI 5928 is a nonwoven web made of 100% polyester (PET) and has a nominal basis weight of 42 gsm, a thickness of 0.46 millimeters, and MD tensile strength of 3.74 Kg cm$^{-1}$ (21 pounds per inch), a CD tensile strength of 1.78 Kg cm$^{-1}$ (10 pounds per inch), a MD elongation at peak load of 33%, and a CD elongation at peak load of 76%. The fabric was coated with PAN, per the above protocol, and activated at 230°C for 30 minutes in a vacuum, yielding a fabric with a 46.4% add-on level. The coating had a golden-bronze color rather than the black color typical of activated carbon.

Another sample (Sample 4-A) of the fabric was coated with PVA, per the above protocol, and activated at 230°C for 30 minutes in a vacuum, yielding a fabric with an add-on level of 61.6%.

Another sample (Sample 4-B) of the fabric was treated with PVA and activated at 170°C for 60 minutes, giving a fabric with a 52% add-on level.

Still another sample of the fabric (sample 4-C) was coated with PVA, per the above protocol, and activated at 170°C for 30 minutes, followed by 2 minutes of washing with a residual acid content of 0.5%. The resulting fabric was black and had an add-on level of 55.8%. The fabric showed good flexibility and durability. Rubbing the fabric with the skin did not result in flakes or black dust coming off the fabric. In a related test, a similar sample was washed for only 1 minute and had a measured residual acid content of 12% and a 55.8% add-on level. Washing a similar sample for 75 seconds resulted in a residual acid content of 6.1%.

Another sample of the fabric (Sample 4-D) was coated with cellulose in DMF, per the above protocol, and activated at 230°C for 30 minutes in a vacuum, yielding a fabric with a 47.5% add-on level with a black color and golden-bronze tinge.

Another sample of the fabric (Sample 4-E) was treated with phenolic resin and activated at 170°C for 60 minutes, yielding a relatively stiff fabric.

Another sample of the fabric (Sample 4-F) was treated with a cellulose solution in DMF, as described above, at 170°C for 60 minutes. The resulting fabric was flexible and had an add-on level of 109%.

## EXAMPLE 5

A portion of reticulated "heavy duty" foam was applied with activated carbon. The foam was taken from a sponge-foam composite distributed by EGL Homecare of Essex, England under the name, the Fairy™ Flipper Combination General Purpose Kitchen Sponge. The sponge portion was cut from the product and treated with PAN and activated at 275°C for 45 minutes. The resulting substrate was black and had an add-on level of 23.8%. The material was relatively stiff and some cells that had previously been open in the foam were occluded by black film.

In another run, the foam was treated with PVA under the same activation conditions, yielding a foam with 26.7% activated carbon (add-on level of 36.4%). The resulting substrate was relatively stiff.

## EXAMPLE 6

Conventional nylon stockings were coated with PAN and activated at 170°C for 30 minutes, giving a relatively stiff material.

## EXAMPLE 7

The abrasive cover of a ScotchBrite™ Dobie® Cleaning Pad (manufactured by 3M (St. Paul, Minnesota)) was coated with activated carbon. The substrate had a melting point of 248°C. The substrate was treated with phenolic resin and activated at 225°C for 45 minutes, resulting in an add-on level of 38%. The substrate was relatively stiff.

The same substrate was also treated with PAN at the same activation conditions, resulting in an add-on level of 7.5%. The substrate was relatively stiff.

The same substrate was also treated with PVA at the same activation conditions, resulting in an add-on level of 4%. The substrate was relatively stiff.

## EXAMPLE 8

A cotton T-shirt was cut into rectangular samples and coated with PAN, per the above protocol, and activated at 250°C for 45 minutes. The resulting substrate was black and relatively stiff. The substrate did not readily stretch under light loading. After pulling gently on the substrate by hand, many of the adjacent woven elements were separated without causing pieces of activated carbon to fall off. As a result, the substrate became elastically extensible. When pulled, light readily shined through the substrate. However, releasing tension allowed the fabric to contract again and return to high opacity.

The substrate was also treated with PVA in one run and phenolic resin in another at the same activation conditions. The resulting substrates were relatively stiff.

The substrate was also treated with cellulose in DMF at the same activation conditions. The resulting substrates were relatively flexible.

## EXAMPLE 9

A meltblown sample of Dow Questra™ fiber (melting point of 264°C), provided by Dow, Inc., was treated with PVA per the above protocol and heated at 240°C for 45 minutes. The resulting substrate was black and had an add-on level of 73.3%. The substrate was relatively stiff.

## EXAMPLE 10

A meltblown web formed from polybutylene terephthalate (PBT) was provided that had a basis weight of about 40 grams per square meter. The substrate was coated with the cellulosic solution in DMF, as described above, and activated at 195°C for 45 minutes. The resulting substrate was relatively flexible and had an add-on level of 61.3%. The substrate did not have openings sufficient to allow light to readily pass through the sample, but it appeared substantially opaque.

Another sample was treated under similar conditions, but with an add-on level of 122%. Samples were also treated with PVA, PAN, and phenolic resin under the same activation conditions (195°C for 45 minutes), yielding fabrics that were relatively stiff.

## EXAMPLE 11

The PGI 5928 PET meltblown fabric of Example 4 was coated with PVA and activated at 230°C for 30 minutes under vacuum, following the procedure used for Sample 4-A in Example 4 but with slightly less added coating, giving a sample with an add-on level of 43.9%. The sample was relatively stiff.

## EXAMPLE 12

A blue metallicized abrasive cover was taken from the Spontex® Flash All Purpose Scouring Pad, which is available from Spontex, Inc., Columbia, Tennessee. The substrate was treated with PAN according to the protocol above, except that PAN was applied using an airbrush and activated at 225°C for 45 minutes. The resulting substrate was black and bronze-colored and had an add-level of 4.6%. The previously blue metallicized portions tended to flake off readily, yielding flakes that were metallic (apparently aluminum-containing) on one side and dark black on the other side.
In another run, the substrate (the Spontex® cover) was coated with PVA by airbrush and activated at 225°C for 45 minutes, resulting in a substrate that had an add-on level of 6.4% with good integrity.
In another run, the substrate was coated with phenolic resin and activated at 225°C for 45 minutes, resulting in a substrate that had an add-on level of 33%. The substrate was relatively stiff.

## EXAMPLE 13

An airlaid mat comprising 92 wt.% bleached kraft softwood fibers and about 8 wt.% bicomponent binder fibers (polyethylene sheath / polyester core) was provided. The mat had a basis weight of about 95 grams per square meter. The substrate was coated with PVA per the above protocol, except that the PVA was applied using an airbrush and activated at 200°C for 30 minutes, yielding a bulky, flexible airlaid substrate having an add-on level of about 8.7%. Many portions of the resulting black mat appeared to have excellent penetration of the solution prior to activation such that the internal portions of the mat displayed black fibers as well as the outer portions. The mat maintained good porosity and allowed light to pass through numerous pores when viewed against a bright light source.
Air permeability was measured with a FX 3300 Air Permeability device manufactured by Textest AG (Zürich, Switzerland), set to a pressure of 125 Pascals with a 7-cm diameter opening. In testing, rather than place the substrate directly over the opening and risk the remote possibility of loose particles entering the vacuum chamber of the device, the substrate was instead tested as it rested on two layers of a single-ply tissue (Scott® handtowel). The two superposed tissue layers were tested for permeability and gave a reading of 2.07 $m^3$ (73.3 cubic feet) per minute. When the substrate was placed over the tissue, the measured permeability of the substrate and tissue layers was 1.48 $m^3$ per minute (52.4 cfm). When the substrate was removed and a third tissue layer added instead, the three tissue layers gave an air permeability of 1.44 $m^3$ per minute (50.9 cfm), which suggested that the air permeability of the substrate was greater than that of a single tissue layer. A single tissue layer had an air permeability of 4.02 m3 per minute (142 cfm).
A similar airlaid mat was treated with a higher level of applied coating (PVA) and activated at 170°C for 30 minutes, resulting in an add-on level of 19.9%. The mat was slightly stiff. A similar mat was treated under the same conditions but with less coating, resulting in an add-on level of 8.5% and a relatively flexible mat. in another run, an airlaid web treated with PVA was activated at 260°C for 30 minutes, resulting in a relatively stiff web that had an add-on level of 7.9%.

## EXAMPLE 14

Dura-Glass 7529 (available from Johns Manville), a nonwoven fiberglass mat made from an E-glass material was treated with activated carbon. Dura-Glass 7529 had a basis weight of 90 grams per square meter and included fibers with a nominal 10-micron fiber diameter. The substrate had a thickness of 0.7 mm, a MD tensile strength greater than 5.9 Kg/cm (100 Ibs/3-inches), a CD tensile strength greater than 5.9 Kg/cm) (100 Ibs/3-inches), and an air permeability of 16.27 $m^3$/M (575 CFM/sq. ft.)
The glass mat was coated with the phenolic coating per the above protocol and activated at 350°C for 30 minutes, yielding a substrate with an add-on level of 116%. The substrate allowed light to pass through in numerous low-basis weight regions that were not occluded by the coating, maintaining good porosity and permeability. Air permeability was measured in the same manner described above in Example 13. The fiberglass-based substrate, when placed on two tissue layers, gave an aggregate air permeability of 1.56 $m^3$/M (55.4 cfm). When the substrate was placed on a single tissue layer, the aggregate air permeability was 2.41 $m^3$/M (85.4 cfm), which suggested that the air permeability of the substrate was substantially greater than that of a single tissue layer. A related sample was prepared using cellulose with zinc chloride to coat the fiberglass, activated at 350°C for 30 minutes, yielding a fabric with 50.5% carbon (over 100% add-on). Another related sample was prepared using PAN as well under the same activation conditions, yielding a fabric with 27% added solids (37% add-on).

## EXAMPLE 15

A polymeric web, termed Nylon Mesh B, was taken from the cover material of the Nylon Scouring Soap Pad of Arden Companies, Southfield Michigan, product M6030. The Nylon Mesh B was treated with PVA and activated at 170°C for 30 minutes. Treatment with PAN followed by activation at 225°C for 45 minutes, yielding a slightly stiff substrate with an add-on level of 1%.

## EXAMPLE 16

A high bulk, fibrous polymeric abrasive material was taken from the Quickie® Light-duty Tub and Tile Scrubber, product number 205 of Quickie Manufacturing Corporation (Cinnaminson, New Jersey). The white polymeric material had an apparent melting point of 212°C. The substrate was coated with PVA, per the protocol above, and activated at 185°C for 45 minutes, yielding a highly porous material with an add-on level of 22.5%. The substrate was slightly stiff and had clumps of activated carbon along the fibers. A similar experiment but using phenolic resin coating gave an add-on level of 46.2%. Another similar run was executed but with a PAN coating, yielding an activated carbon with 11 weight % added activated carbon coating.

## EXAMPLE 17

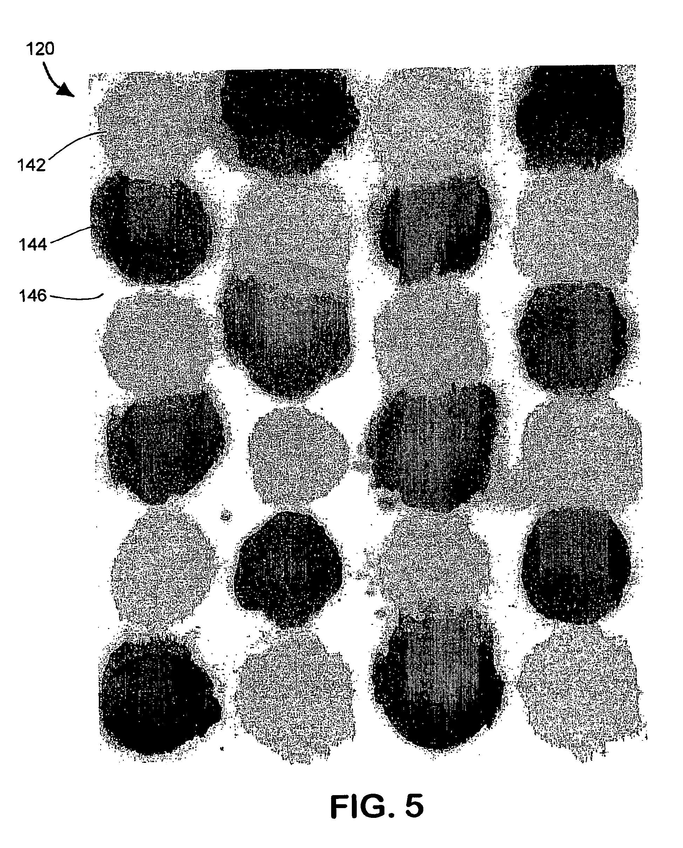

To illustrate the ability of the present invention to create heterogeneous activated carbon fabrics having nonuniform patterns of activated carbon or patterns of two or more kinds of activated carbon on a single substrate, a PET fabric as described in Example 4 (the PGI 5928 fabric was treated with both PAN and PVA solutions according to the protocols B and C above). Application was by spray painting, with some brush application by hand to adjust the patterns. In a first example, a staggered array of roughly 1-inch circles of the treated areas was placed on an 8-inch by 11-inch section of the PET fabric. The result was about 50% of the fabric being covered with treated regions. The fabric was activated at 170°C for 30 minutes, yielding black circles for the PVA-treated regions and golden circles for the PAN-treated regions in this sample (Sample 1), in a staggered array having a total of about 22 weight % activated carbon solids added, with a white background corresponding to the untreated regions around the treated circles. Figure 5 is a grayscale image of the treated sample 120, showing the golden-colored regions 142 corresponding to PAN treatment, the black regions 144 corresponding to PVA treatment, and white untreated regions 146.

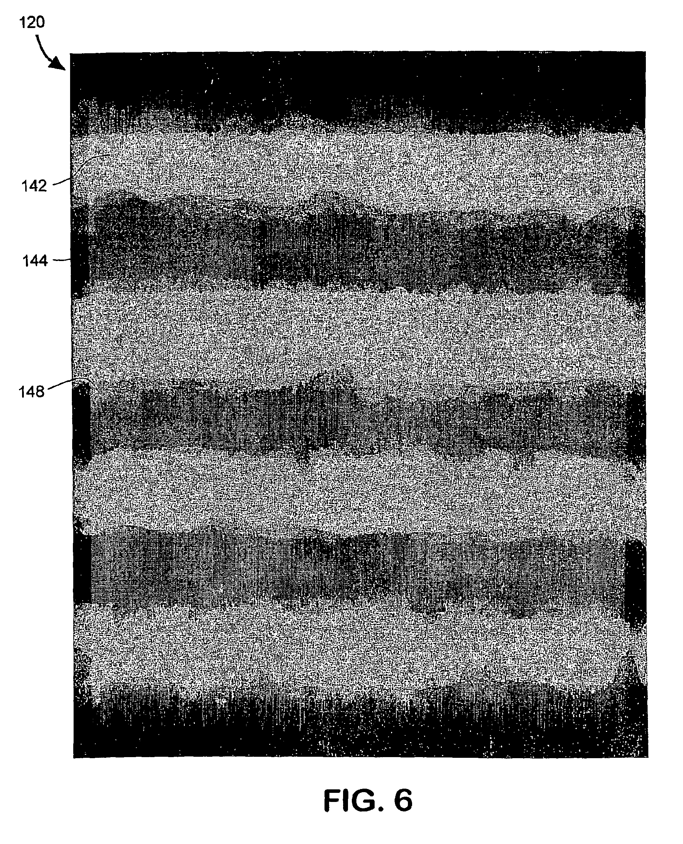

A second 20.3 cm (8-inch) by 27.94 cm (11-inch) section of the PET fabric was treated in the same manner but with alternating stripes of PVA-treated and PAN-treated regions. After activation under the same conditions as Sample 1, the sample (Sample 2) displayed alternating bands of black and golden regions corresponding, respectively, to the PVA-treated and PAN-treated regions, with substantially no untreated portions on the fabric. Figure 6 is a grayscale image of the treated sample 120, showing the golden-colored regions 142 corresponding to PAN treatment, the black regions 144 corresponding to PVA treatment, and dual-treatment regions 148 where both PVA and PAN solutions had been applied (regions of overlap).

## EXAMPLE 18

An activated carbon fabric was prepared from a 150 gsm extruded mesh of EVA (ethylene vinyl acetate) having a thickness of 0.5 mm and approximately 50% open area with round holes about 0.5 mm in diameter arrayed in a staggered bilateral array. The web had elastic properties similar to a conventional rubber. The mesh was dip coated with PVA and activated at 170°C for 30 minutes, changing the initially white color of the mesh to black from the activated carbon coating. The mesh maintained a stretchable, elastic characteristic.

## TEST RESULTS FOR EXAMPLES

As indicated above, the ability to form substrates with a durable activated carbon coating was demonstrated. In many instances, the resulting activated carbon substrates were also relatively flexible. However, even for the substrates that appeared to be relatively stiff, it should be understood that flexibility could be readily imparted by varying the activation temperature, activation time period, add-on level, type of polymeric material and/or activation agent, as well as other parameters discussed above.

For several of the examples above, the ability of the activated carbon to reduce odor was also tested. Specifically, odor reduction was measured using "Headspace Gas Chromatography", which is described in detail below.

### Headspace Gas Chromatography

Adsorption testing was done with a headspace gas chromatography (headspace GC) procedure to measure the amount of an odoriferous compound removed from the gas phase by activated carbon materials. The headspace GC testing was conducted on an Agilent Technologies 5890, series II gas chromatograph with an Agilent Technology 7694 headspace sampler (Agilent Technologies, Waldbronn, Germany). Helium was used as the carrier gas (injection port pressure: 8.7 x 10$^4$ Nm$^{-2}$ (12.7 psig); headspace vial pressure: 1.08 x 10$^5$ Nm$^{-2}$ (15.8 psig; supply line pressure is at 4.13 x 10$^5$ Nm$^{-2}$ (60 psig)). For ammonia (NH$_3$), a HayeSep P stainless steel column (Alltech Associates, Inc. of Deerfield, Illinois) was used that had a 60/80 mesh, a length of 2.43 m (8 feet), and an outer diameter of 0.317 cm (1/8 inch). For triethylamine (TEA), trimethylamine (TMA), dimethyldisulphide (DMDS), and ethyl mercaptan, a DB-624 column (J&W Scientific, Inc. of Folsom, California) was used that had a length of 30 meters, an internal diameter of 0.25 millimeters, and a 1.4-micron film.

The operating parameters for the headspace GC, as a function of the odoriferous agent being tested, are shown below in Table 1:

**Table 1. Operating Parameters for the Headspace GC Device.**

| Headspace Parameters | | Values | |
|---|---|---|---|
| | | TMA, TEA, DMDS | NH$_3$ |
| Zone Temps, °C | Oven | 37 | 37 |
| | Loop | 85 | 42 |
| | TR. Line | 90 | 47 |
| Event Time, minutes | GC Cycle time | 10.0 | 10.0 |
| | Vial eq. Time | 10.0 | 10.0 |
| | Pressuriz. Time | 0.20 | 0.20 |
| | Loop fill time | 0.20 | 0.20 |
| | Loop eq. Time | 0.15 | 0.15 |
| | Inject time | 0.30 | 0.30 |
| Vial Parameters | First vial | 1 | 1 |
| | Last vial | 1 | 1 |
| | Shake | [off] | [off] |

The test procedure involved placing about 0.14 grams of the activated carbon material (unless otherwise specified) inside a 20-cubic centimeter headspace vial. The amount of sample can be adjusted to keep the measurement within range of the instrument for better accuracy. Using a syringe, an aliquot of an odoriferous agent was also placed in the vial, taking care not to let the liquid and activated carbon contact. The vial was then sealed with a cap and septum and placed in the headspace GC oven at a temperature of 37°C. After ten minutes, a hollow needle was inserted through the septum and into the vial. A 1-cubic

**Table 2. Headspace Gas Chromatography Results: Adsorption of Ammonia**

| Sample | Ret Time (min) | Area (counts*s) | mg/g fabric | % Removed | Fabric Mass (g) | mg/g a.c. | Carbon mass (g) |
|---|---|---|---|---|---|---|---|
| Control | | | | | | | |
| 6uL NH3 - Control 1 | 1.224 | 1.30E+06 | | | | | |
| 6uL NH3 - Control 2 | 1.217 | 1.46E+06 | | | N / A | | |
| 6uL NH3 - Control 3 | 1.213 | 1.48E+06 | | | | | |
| Average | 1.218 | 1.41 E+06 | | | | | |
| St Dev | 0.006 | 9.87E+04 | | | | | |

(continued)

| | | | | | | | |
|---|---|---|---|---|---|---|---|
| E-Glass Cellulose, 50.5% carbon (Example 14) | | | | | | | |
| Rep 1 | 1.309 | 2.48E+05 | 17.2 | 82.45% | 0.0718 | 34.1 | 0.0363 |
| Rep 2 | 1.289 | 3.60E+05 | 16.5 | 74.53% | 0.0679 | 32.6 | 0.0343 |
| Average | 1.299 | 3.04E+05 | 16.8 | 78.49% | 0.0699 | 33.4 | 0.0353 |
| St Dev | 0.014 | 7.92E+04 | 0.54 | 5.60% | 0.0028 | 0.8 | 0.0014 |
| PET w/ PVA: activated 230C/30 min/vacuum/38.1 % carbon (Example 4) | | | | | | | |
| Rep 1 - tested as-is | 1.437 | 2.62E+04 | 16.1 | 98.15% | 0.0914 | 42 | 0.0347 |
| Rep 2 - dried 1 hour @ 100C | 1.426 | 2.82E+04 | 16.3 | 98.00% | 0.0902 | 43 | 0.0343 |
| Average | 1.432 | 2.72E+04 | 16.2 | 98.08% | 0.0908 | 43 | 0.0345 |
| St Dev | 0.008 | 1.41 E+03 | 0.13 | 0.10% | 0.00 | 0.35 | 0.0003 |
| PET w/ PVA; activated 230C/30 min/vacuum/30.5% carbon in foil (Example 11) | | | | | | | |
| Rep 1 - tested as-is | 3.886 | 9.23E+05 | 5.7 | 34.69% | 0.0907 | 18.8 | 0.0277 |
| Rep 2 - dried @ 100C/1 hour | 3.880 | 1.56E+06 | NA | NA | 0.0893 | NA | 0.0272 |

**Table 3. Headspace Gas Chromatography Results: Adsorption of Ammonia**

| Sample | Ret Time (min) | Area (counts*s) | mg/g fabric | % Removed | Fabric Mass (g) | mg/g a.c. | Carbon mass (g) |
|---|---|---|---|---|---|---|---|
| 6uL NH3 - Control 1 | 1.216 | 1.27E+06 | | | | | |
| 6uL NH3 - Control 2 | 1.209 | 1.44E+06 | | | N / A | | |
| 6uL NH3 - Control 3 | 1.210 | 1.41 E+06 | | | | | |
| Average | 1.212 | 1.37E+06 | | | | | |
| St Dev | 0.004 | 9.07E+04 | | | | | |
| Scotch Brite PVA w/ H3P04, 11% (Example 3) | | | | | | | |
| Sample 2 - Rep 1 | 1.256 | 5.87E+05 | 6.16 | 57.26% | 0.1395 | 37.8 | 0.0227 |
| Sample 2 - Rep 2 | 1.257 | 5.42E+05 | 6.51 | 60.53% | 0.1394 | 40.0 | 0.0227 |

(continued)

| | | | | | | | |
|---|---|---|---|---|---|---|---|
| Scotch Brite PVA w/ H3P04, 11% (Example 3) | | | | | | | |
| Average | 1.257 | 5.65E+05 | 6.34 | 58.90% | 0.1395 | 38.9 | 0.0227 |
| St Dev | 0.001 | 3.18E+04 | 0.25 | 2.32% | 0.0001 | 1.55 | 0 |
| E-Glass Cellulose, 50.5% carbon (Example 14) | | | | | | | |
| Sample 3 - Rep 1 | 1.324 | 1.83E+05 | 9.35 | 86.67% | 0.1391 | 18.7 | 0.0696 |
| Sample 3 - Rep 2 | 1.338 | 1.42E+05 | 9.41 | 89.66% | 0.1429 | 18.8 | 0.0715 |
| Average | 1.331 | 1.63E+05 | 9.38 | 88.17% | 0.1410 | 18.8 | 0.0705 |
| St Dev | 0.010 | 2.90E+04 | 0.05 | 2.11% | 0.0027 | 0.09 | 0.0013 |
| Spontex Flash PVA w/ H3P04 (Example 12) | | | | | | | |
| Sample 6 - Rep 1 | 1.240 | 7.51 E+05 | 4.8 | 45.32% | 0.1422 | 79.7 | 0.0085 |
| Sample 6 - Rep 2 | 1.266 | 4.99E+05 | 6.7 | 63.67% | 0.1418 | 112.2 | 0.0085 |
| Average | 1.253 | 6.25E+05 | 5.8 | 54.49% | 0.1420 | 95.9 | 0.0085 |
| St Dev | 0.018 | 1.78E+05 | 1.38 | 12.98% | 0.0003 | 23.03 | 0 |

centimeter sample of the headspace (air inside the vial) was then injected into the headspace GC. Initially, a control vial with only the aliquot of odoriferous agent (no activated carbon) was tested to define 0% odoriferous agent adsorption. To calculate the amount of headspace odoriferous agent removed by the activated carbon, the peak area for the odoriferous agent from the vial with activated carbon is compared to the peak area from the odoriferous agent control vial (no activated carbon).

Using the aforementioned test, the odor reduction of the samples formed in Examples 3-4, 11-12, and 14-16 were tested. The results are set forth below:

**Table 4. Headspace Gas Chromatography Results: Adsorption of DMDS**

| Sample | Ret Time (min) | Area (counts*s) | mg/g fabric | % Removed | Fabric Mass (g) | mg/g a.c. | Carbon mass (g) |
|---|---|---|---|---|---|---|---|
| Control | | | | | | | |
| 3.6uL DMDS - Control 1 | 3.867 | 5.98E+06 | | | | | |
| 3.6uL DMDS - Control 2 | 3.866 | 5.97E+06 | | | N / A | | |
| 3.6uL DMDS - Control 3 | 3.864 | 5.96E+06 | | | | | |
| Average | 3.866 | 5.97E+06 | | | | | |
| St Dev | 0.002 | 1.00E+04 | | | | | |

(continued)

| | | | | | | | |
|---|---|---|---|---|---|---|---|
| E-Glass Cellulose, 50.5% carbon (Example 14) | | | | | | | |
| Rep 1 | 3.891 | 4.87E+05 | 48 | 91.84% | 0.0720 | 96.2 | 0.0360 |
| Rep 2 | 3.889 | 6.09E+05 | 49 | 89.80% | 0.0687 | 98.6 | 0.0344 |
| Average | 3.890 | 5.48E+05 | 49 | 90.82% | 0.0704 | 97.3 | 0.0352 |
| St Dev | 0.001 | 8.63E+04 | 0.8 | 1.45% | 0.0023 | 1.7 | 0.0012 |
| PET w/ PVA: activated 230C/30 min/vacum/38.1% carbon (Example 4) | | | | | | | |
| Rep 1 - tested as-is | 1.437 | 2.62E+04 | 41 | 99.56% | 0.0914 | 108.1 | 0.0347 |
| Rep 2 - dried 1 hour @ 100c | 1.426 | 2.82E+04 | 42 | 99.53% | 0.0902 | 109.5 | 0.0343 |
| Average | 1.432 | 2.72E+04 | 41 | 99.54% | 0.0908 | 108.8 | 0.0345 |
| St Dev | 0.008 | 1.41 E+03 | 0.4 | 0.02% | 0.0008 | 1.0 | 0.0003 |
| Control: Calgon RGC 40x100 activated carbon particles | | | | | | | |
| Rep 1 - tested as-is | 3.880 | 1.91 E+06 | 610 | 68.01% | 0.0042 | 610.4 | 0.0042 |
| Rep 2 - dried @ 100C/1 hour | 3.882 | 1.37E+06 | 593 | 77.05% | 0.0049 | 592.8 | 0.0049 |
| Average | 3.881 | 1.64E+06 | 601 | 72.53% | 0.0046 | 601.0 | 0.0046 |
| St Dev | 0.001 | 3.82E+05 | 12.5 | 6.40% | 0.0005 | 12.5 | 0.0005 |
| PET w/ PVA; activated 230C/30 min/vacuum/30.5% carbon in foil (Example 11) | | | | | | | |
| Rep 1 - tested as-is | 3.886 | 9.23E+05 | 35 | 84.54% | 0.0907 | 115.2 | 0.0277 |
| Rep 2 - dried @ 100C/1 hour | 3.880 | 1.56E+06 | 31 | 73.87% | 0.0893 | 102.2 | 0.0272 |
| Average | 3.883 | 1.24E+06 | 33 | 79.20% | 0.0900 | 108.8 | 0.0275 |
| St Dev | 0.004 | 4.50E+05 | 2.8 | 7.54% | 0.0010 | 9.2 | 0.0003 |
| PET w/ Cellulose; activated 230C/30 min/vacuum/ 32.3% Carbon (Example 4) | | | | | | | |
| Rep 1 - tested as-is | 3.864 | 4.55E+06 | 10 | 23.79% | 0.0922 | 30.1 | 0.0298 |

(continued)

| PET w/ Cellulose; activated 230C/30 min/vacuum/ 32.3% Carbon (Example 4) | | | | | | | |
|---|---|---|---|---|---|---|---|
| Rep 2 - dried @ 100C/1 hour | 3.860 | 5.73E+06 | 2 | 4.02% | 0.0919 | 5.1 | 0.0297 |
| Average | 3.862 | 5.14E+06 | 6 | 13.90% | 0.0921 | 17.6 | 0.0297 |
| St Dev | 0.003 | 8.34E+05 | 5.7 | 13.98% | 0.0002 | 17.7 | 0.0001 |
| PET w/ PAN; activated 230C/30 min/vacuum/31.7% Carbon (Example 4) | | | | | | | |
| Rep 1 - tested as-is | 3.861 | 5.75E+06 | 2 | 3.69% | 0.0896 | 4.9 | 0.0284 |
| Rep 2 - dried @ 100C/1 hour | 3.861 | 5.85E+06 | 1 | 2.01% | 0.0917 | 2.6 | 0.0291 |
| Average | 3.861 | 5.80E+06 | 1 | 2.85% | 0.0907 | 3.7 | 0.0287 |
| St Dev | 0.000 | 7.07E+04 | 0.5 | 1.18% | 0.0015 | 1.6 | 0.0005 |

**Table 5A. Headspace Gas Chromatography Results: Adsorption of DMDS**

| Sample | Ret Time (min) | Area (counts*s) | mg/g fabric | % Removed | Fabric Mass (g) | mg/g a.c. | Carbon mass (g) |
|---|---|---|---|---|---|---|---|
| Control | | | | | | | |
| 3.6uL DMDS - Control 1 | 3.888 | 6.04E+06 | | | | | |
| 3.6uL DMDS - Control 2 | 3.889 | 6.06E+06 | | | N / A | | |
| 3.6uL DMDS - Control 3 | 3.892 | 6.11 E+06 | | | | | |
| Average | 3.890 | 6.07E+06 | | | | | |
| St Dev | 0.002 | 3.61 E+04 | | | | | |
| Scrub Layer A Phenolic w/ ZnCl2, 31.6% (Example 16) | | | | | | | |
| Sample 1 - Rep 1 | 3.890 | 4.99E+06 | 4.7 | 17.79% | 0.1420 | 14.9 | 0.0449 |
| Sample 1 - Rep 2 | 3.893 | 4.40E+06 | 7.5 | 27.51% | 0.1387 | 23.7 | 0.0438 |
| Average | 3.892 | 4.70E+06 | 6.1 | 22.65% | 0.1404 | 19.3 | 0.0444 |
| St Dev | 0.002 | 4.17E+05 | 1.9 | 6.87% | 0.0023 | 6.2 | 0.0007 |

(continued)

| | | | | | | |
|---|---|---|---|---|---|---|
| Scotch Brite PVA w/ H3P04. 11 % (Example 3) | | | | | | |
| Sample 2 - Rep 1 | 3.898 | 3.34E+06 | 12.3 | 44.98% | 0.1384 | 75.2 | 0.0226 |
| Sample 2 - Rep 2 | 3.900 | 3.17E+06 | 12.8 | 47.78% | 0.1405 | 78.6 | 0.0229 |
| Average | 3.899 | 3.26E+06 | 12.5 | 46.38% | 0.1395 | 76.9 | 0.0227 |
| St Dev | 0.001 | 1.20E+05 | 0.4 | 1.98% | 0.0015 | 2.5 | 0.0002 |
| E-Glass Cellulose, 50.5% carbon (Example 14) | | | | | | |
| Sample 3 - Rep 1 | 3.920 | 7.12E+04 | 26.6 | 98.83% | 0.1401 | 52.7 | 0.0708 |
| Sample 3 - Rep 2 | 3.921 | 7.44E+04 | 26.6 | 98.77% | 0.1398 | 52.7 | 0.0706 |
| Average | 3.921 | 7.28E+04 | 26.6 | 98.80% | 0.1400 | 52.7 | 0.0707 |
| St Dev | 0.001 | 2.26E+03 | 0.0 | 0.04% | 0.0002 | 0.1 | 0.0001 |
| E-Glass PAN w/ ZnCl2 (Example 14) | | | | | | |
| Sample 4 - Rep 1 | 3.909 | 1.44E+06 | 20.1 | 76.28% | 0.1433 | 74.3 | 0.0387 |
| Sample 4 - Rep 2 | 3.909 | 1.88E+06 | 18.2 | 69.03% | 0.1430 | 67.4 | 0.0386 |
| Average | 3.909 | 1.66E+06 | 19.1 | 72.65% | 0.1432 | 70.9 | 0.0387 |
| St Dev | 0.000 | 3.11 E+05 | 1.3 | 5.13% | 0.0002 | 4.9 | 0.0001 |
| E-Glass PAN w/ ZnCl2 & DMF (Example 14) | | | | | | |
| Sample 5 - Rep 1 | 3.896 | 4.31 E+06 | 8.0 | 29.00% | 0.1370 | 28.5 | 0.0384 |
| Sample 5 - Rep 2 | 3.900 | 3.19E+06 | 12.7 | 47.45% | 0.1405 | 45.5 | 0.0393 |
| Average | 3.898 | 3.75E+06 | 10.4 | 38.22% | 0.1388 | 37.1 | 0.0389 |
| St Dev | 0.003 | 7.92E+05 | 3.4 | 13.05% | 0.0025 | 12.0 | 0.0007 |

### Table 5B. Headspace Gas Chromatography Results: Adsorption of DMDS

| Sample | Ret Time (min) | Area (counts*s) | mg/g fabric | % Removed | Fabric Mass (g) | mg/g a.c. | Carbon mass (g) |
|---|---|---|---|---|---|---|---|
| Control | | | | | | | |
| 3.6uL DMDS - Control 1 | 3.888 | 6.04E+06 | | | | | |
| 3.6uL DMDS - Control 2 | 3.889 | 6.06E+06 | | | N / A | | |
| 3.6uL DMDS - Control 3 | 3.892 | 6.11 E+06 | | | | | |
| Average | 3.890 | 6.07E+06 | | | | | |
| St Dev | 0.002 | 3.61 E+04 | | | | | |
| Spontex Flash PVA w/ H3P04 (Example 12) | | | | | | | |
| Sample 6 - Rep 1 | 3.899 | 3.64E+06 | 10.7 | 40.03% | 0.1407 | 178.8 | 0.0084 |
| Sample 6 - Rep 2 | 3.898 | 4.07E+06 | 8.9 | 32.95% | 0.1400 | 147.9 | 0.0084 |
| Average | 3.899 | 3.86E+06 | 9.8 | 36.49% | 0.1404 | 163.4 | 0.0084 |
| St Dev | 0.001 | 3.04E+05 | 1.3 | 5.01% | 0.0005 | 21.8 | 0.0000 |
| Nylon Mesh B + PAN (Example 15) | | | | | | | |
| Sample 7 - Rep 1 | 3.893 | 5.81 E+06 | 1.2 | 4.28% | 0.1396 | 115.7 | 0.0014 |
| Sample 7 - Rep 2 | 3.894 | 5.76E+06 | 1.3 | 5.11% | 0.1430 | 134.6 | 0.0014 |
| Average | 3.894 | 5.79E+06 | 1.3 | 4.70% | 0.1413 | 125.3 | 0.0014 |
| St Dev | 0.001 | 3.54E+04 | 0.1 | 0.58% | 0.0024 | 13.4 | 0.0000 |

Table 2 shows headspace gas chromatography results for the adsorption of ammonia for materials from Examples 4, 11, and 14 above. The first column shows the sample being tested. The second column is the area (counts * seconds). Calibration results are shown for three control runs consisting of 6 microliters of 28% ammonia solution. The average area, 1.41 E+06, is proportional to the amount of ammonia used in the test (6 microliters). When ammonia is adsorbed by adsorbent materials, the area measured for the same initial amount of ammonia placed in the test apparatus will be decreased. For test runs with activated carbon materials, the fourth column shows the percent removed of the odorant, calculated as:

$$\% \text{ removed} = (\text{mean area of the controls} - \text{area of the run in question})/$$

$$\text{mean area of the controls} * 100\%$$

The absolute amount removed is the percent removed multiplied by the amount of odorant injected, which was 1.5 milligrams. The absolute amount removed divided by the fabric sample mass is given in the third column as "mg/g fabric." When the amount removed is divided by the mass of the activated carbon coating on the fabric, the result is milligrams of odorant adsorbed / activated carbon mass, given under the heading "mg/g a.c." in the sixth column. For example, in Table 2, Repetition 1 ("Rep. 1") of the activated carbon fabric from Example 14 was made from an E-glass fabric coated with cellulose and chemically converted to activated carbon material. The sample was tested by placing 0.0718 grams of the fabric in the test device, having 0.0363 grams of activated carbon coating. When 6 microliters of ammonia were injected, the measured chromatographic curve had an area of 2.48E+05. The mean area of the three control runs was 1.41E+06. The percentage of the 6 microliters of ammonia removed by the sample is (1.41E+06 - 2.48E+05)/ 1.41E+06 * 100% = 82.4%. The mg/g a.c. value is 0.824*1.5 mg/36.3 mg = 34.1 mg/ g a.c.

Table 3 shows additional testing for materials of Examples 3, 12, and 14. Of the results for ammonia adsorption, those for PET treated with PVA showed the highest removal of ammonia, with about 98% removal being observed. However, Example 12, with relatively little activated carbon (0.0085 g of activated carbon in the 0.14 g samples - see the columns labeled "Carbon mass" and "Fabric mass"), gave the highest adsorption in terms of mg of ammonia adsorbed per gram of activated carbon material, with a mean of about 96 mg/g a.c.

Table 4 shows test results for adsorption of DMDS by materials from Examples 4, 11, and 14, as well as commercially available activated carbon granules in the form of Calgon RGC 40 x 1000 activated carbon marketed by Calgon Carbon, Inc. (Pittsburgh, PA). The data for the commercial activated carbon granules shows an unusually high level of adsorption because of the much smaller amount of activated carbon that was employed in the test. Other related tests with larger amounts of activated carbon granules are expected to give adsorption levels around 100 to 150 mg/g for commercial activated carbon granules.

Tables 5A and 5B, respectively, show additional results from headspace GC testing for DMDS adsorption by materials from Examples 3, 14, and 16. The cellulose-treated fiberglass of Example 14 and the PVA-treated commercial scrub pad (Example 3) gave the highest levels of adsorption.

It should be recognized that the pore structure and surface chemistry of any one kind of activated carbon material or an activated carbon fabric may not be suitable for all odorants, and low adsorption of one or more odorants may be compensated in commercial practice by excellent adsorption of other odorants or by other benefits such as improved strength, durability, or flexibility.

## Claims

1. A flexible substrate embodied in an absorbent article (10) in the form of a sanitary napkin, diaper, diaper pant, feminine napkin or children's training pant, **characterized in that** the substrate is applied with an activated carbon coating, said activated carbon coating being formed from a polymeric material and an activation agent heated to an activation temperature of from about 100°C to about 300°C, wherein the flexible substrate contains a stretchable component that, upon application of a force, is stretchable to a stretched, biased length that is at least about 120% its unstretched length.

2. A flexible substrate as defined in claim 1, wherein said stretchable component includes an elastomeric polymer.

3. A flexible substrate as defined in claim 2, wherein the softening point of said elastomeric polymer is greater than said activation temperature.

4. A flexible substrate as defined in claim 3, wherein said elastomeric polymer is selected from the group consisting of fluoropolymers; perfluoropolymers; highly saturated nitrile polymers; ethylene vinyl acetate polymers, silicone polymers; polyacrylate elastomers; and combinations thereof.

5. A flexible substrate as defined in claim 2, wherein said elastomeric polymer is contained within an elastic laminate.

6. A flexible substrate as defined in claim 5, wherein said elastic laminate is selected from the group consisting of neck-bonded laminates, stretch-bonded laminates, necked stretched-bonded laminates, and combinations thereof.

7. A flexible substrate as defined in claim 1, wherein said stretchable component contains a polymeric mesh.

8. A flexible substrate as defined in claim 7, wherein said polymeric mesh is selected from the group consisting of polyester meshes, nylon meshes, and combinations thereof.

9. A flexible substrate as defined in any preceding claim, wherein said polymeric material is selected from the group consisting of polyacrylonitrile, phenolic resins, ethylene vinyl acetate or copolymers thereof, polyvinyl alcohol, cellulose or other natural or synthetic polysaccharides, cellulose derivatives or other polysaccharide derivatives, polystyrene, polypropylene, polyvinyl chloride, polymethacrylates, polymethacrylic acids, polylactic acid, and combinations thereof.

10. A flexible substrate as defined in any preceding claim, wherein said activation agent comprises a compound selected from the group consisting of acids, metal halides, hydroxides, and combinations thereof.

11. A flexible substrate as defined in any preceding claim, wherein said polymeric material and said activation agent are heated to a temperature of from about 170°C to about 300°C.

12. A flexible substrate as defined in any preceding claim, wherein said substrate contains a nonwoven fabric.

13. A flexible substrate as defined in any preceding claim, wherein the add-on level of said activated carbon coating is from about 5% to about 50% of the mass of said substrate.

14. A flexible substrate as defined in any preceding claim, wherein said activated carbon coating is applied in a preselected pattern on a first surface of said substrate.

15. A flexible substrate as defined in any preceding claim, wherein said activated carbon coating comprises less than 100% of at least one surface of said substrate.

16. A flexible substrate as defined in claim 15, wherein said activated carbon coating comprises from about 20% to about 60% of at least one surface of said substrate.

17. A flexible substrate as defined in any preceding claim, wherein said substrate has an average pore size of from about 0.1 millimeters to about 10 millimeters.

18. A flexible substrate as defined in claim 17, wherein said substrate has an average pore size of from about 0.3 millimeters to about 5 millimeters.

19. A flexible substrate as claimed in claim 1, wherein the activation agent is heated to an activation temperature of from about 170°C to about 300°C, wherein the add-on level of said activated carbon coating is from about 5% to about 50% of the mass of said substrate, said substrate having an average pore size of from about 0.1 millimeters to about 10 millimeters.

20. A flexible substrate as defined in claim 19, wherein said stretchable component includes an elastomeric polymer.

21. A flexible substrate as defined in claim 20, wherein the softening point of said elastomeric polymer is greater than said activation temperature.

22. A flexible substrate as defined in claim 19, 20 or 21, wherein said substrate has an average pore size of from about 0.3 millimeters to about 5 millimeters.

23. A flexible substrate as claimed in any preceding claim, in which an integral coating of activated carbon is formed around fibers of the substrate.

24. A flexible substrate as claimed in any preceding claim, in which the coating forms a network that surrounds substrate material and/or chemical bonds are formed between the coating and the substrate material.

25. A flexible substrate as claimed in any preceding claim, in which the polymeric material is crosslinked.

26. A method of forming a flexible substrate used in an absorbent article (10) in the form of a sanitary napkin, diaper, diaper pant, feminine napkin or children's training pant, **characterized by** providing a substrate having a first surface and a second surface, wherein the flexible substrate contains a stretchable component that, upon application of a force, is stretchable to a stretched, biased length that is at least about 120% its unstretched length;
applying a polymeric material and an activation agent to said first surface of said substrate;
heating said polymeric material and said activation agent to an activation temperature of from about 100°C to about 300°C to form an activated carbon coating; and

incorporating the substrate into a said absorbent article.

27. A method as defined in claim 26, wherein said stretchable component includes an elastomeric polymer.

28. A method as defined in claim 27, wherein the softening point of said elastomeric polymer is greater than said activation temperature.

29. A method as defined in claim 26, 27 or 28, wherein said stretchable component contains a polymeric mesh.

30. A method as defined in any of claims 26 to 29, wherein said polymeric material is selected from the group consisting of polyacrylonitrile, phenolic resins, ethylene vinyl acetate or copolymers thereof, polyvinyl alcohol, cellulose or other natural or synthetic polysaccharides, cellulose derivatives or other polysaccharide derivatives, polystyrene, polypropylene, polyvinyl chloride, polymethacrylates, polymethacrylic acids, polylactic acid, and combinations thereof.

31. A method as defined in any of claims 26 to 30, wherein said activation agent is selected from the group consisting of acids, metal halides, hydroxides, and combinations thereof.

32. A method as defined in any of claims 26 to 31, wherein said polymeric material and said activation agent are heated to a temperature of from about 170°C to about 300°C.

33. A method as defined in any of claims 26 to 32, wherein the add-on level of said activated carbon coating is from about 5% to about 50% of the mass of said substrate.

34. A method as defined in any of claims 26 to 33, wherein said activated carbon coating comprises less than 100% of said first surface of said substrate.

35. A method as defined in any of claims 26 to 33, wherein said substrate has an average pore size of from about 0.1 millimeters to about 10 millimeters.

36. A method as defined in claim 35, wherein said substrate has an average pore size of from about 0.3 millimeters to about 5 millimeters.

37. A method as defined in any of claims 26 to 36, wherein said polymeric material, said activation agent, or combinations thereof, are printed onto said first surface of said substrate.

38. A method as defined in any of claims 26 to 37, wherein a second activated carbon coating is formed on said second surface of said substrate.


**Patentansprüche**

1. Flexibles Substrat, verkörpert in einem absorbierenden Gegenstand (10) in Form einer Hygienebinde, Windel, Windelhose, Damenbinde oder Kindertrainingshose, **dadurch gekennzeichnet, dass** das Substrat mit einer Aktivkohlebeschichtung versehen ist, wobei die Aktivkohlebeschichtung aus einem polymeren Material und einem Aktivierungsmittel, erwärmt auf eine Aktivierungstemperatur von etwa 100 °C bis etwa 300 °C, gebildet ist, worin das flexible Substrat eine verstreckbare Komponente enthält, die bei Anwendung einer Kraft auf eine gestreckte vorgespannte Länge verstreckbar ist, die wenigstens etwa 120 % ihrer ungestreckten Länge beträgt.

2. Flexibles Substrat wie in Anspruch 1 definiert, worin die verstreckbare Komponente ein elastomeres Polymer enthält.

3. Flexibles Substrat wie in Anspruch 2 definiert, worin der Erweichungspunkt des elastomeren Polymers größer ist als die Aktivierungstemperatur.

4. Flexibles Substrat wie in Anspruch 3 definiert, worin das elastomere Polymer ausgewählt ist aus der Gruppe bestehend aus Fluorpolymeren, Perfluorpolymeren, hoch gesättigten Nitrilpolymeren, Ethylen-Vinylacetat-Polymeren, Siliconpolymeren, Polyacrylatelastomeren und Kombinationen davon.

5. Flexibles Substrat wie in Anspruch 2 definiert, worin das elastomere Polymer in einem elastischen Laminat enthalten ist.

6. Flexibles Substrat wie in Anspruch 5 definiert, worin das elastische Laminat ausgewählt ist aus der Gruppe bestehend aus neck-bonded Laminaten, stretch-bonded Laminaten, necked stretched-bonded Laminaten und Kombinationen davon.

7. Flexibles Substrat wie in Anspruch 1 definiert, worin die verstreckbare Komponente ein polymeres Gitter enthält.

8. Flexibles Substrat wie in Anspruch 7 definiert, worin das polymere Gitter ausgewählt ist aus der Gruppe bestehend aus Polyestergittern, Nylongittern und Kombinationen davon.

9. Flexibles Substrat wie in einem der vorhergehenden Ansprüche definiert, worin das polymere Material ausgewählt ist aus der Gruppe bestehend aus Polyacrylnitril, phenolischen Harzen, Ethylen-Vinylacetat oder Copolymeren davon, Polyvinylalkohol, Cellulose oder anderen natürlichen oder synthetischen Polysacchariden, Cellulosederivaten oder anderen Polysaccharidderivaten, Polystyrol, Polypropylen, Polyvinylchlorid, Polymethacrylaten, Polymethacrylsäuren, Polymilchsäure und Kombinationen davon.

10. Flexibles Substrat wie in einem der vorhergehenden Ansprüche definiert, worin das Aktivierungsmittel eine Verbindung umfasst, ausgewählt aus der Gruppe bestehend aus Säuren, Metallhalogeniden, Hydroxiden und Kombinationen davon.

11. Flexibles Substrat wie in einem der vorhergehenden Ansprüche definiert, worin das polymere Material und das Aktivierungsmittel auf eine Temperatur von etwa 170 °C bis etwa 300 °C erwärmt werden.

12. Flexibles Substrat wie in einem der vorhergehenden Ansprüche definiert, worin das Substrat ein nicht gewebtes Erzeugnis enthält.

13. Flexibles Substrat wie in einem der vorhergehenden Ansprüche definiert, worin die Zugabemenge der Aktivkohlebeschichtung etwa 5 % bis etwa 50 % der Masse des Substrats beträgt.

14. Flexibles Substrat wie in einem der vorhergehenden Ansprüche definiert, worin die Aktivkohlebeschichtung in einem vorausgewählten Muster auf eine erste Oberfläche des Substrats aufgebracht ist.

15. Flexibles Substrat wie in einem der vorhergehenden Ansprüche definiert, worin die Aktivkohlebeschichtung weniger als 100 % von wenigstens einer Oberfläche des Substrats umfasst.

16. Flexibles Substrat wie in Anspruch 15 definiert, worin die Aktivkohlebeschichtung etwa 20 % bis etwa 60 % von wenigstens einer Oberfläche des Substrats umfasst.

17. Flexibles Substrat wie in einem der vorhergehenden Ansprüche definiert, worin das Substrat eine mittlere Porengröße von etwa 0,1 Millimeter bis etwa 10 Millimeter hat.

18. Flexibles Substrat wie in Anspruch 17 definiert, worin das Substrat eine mittlere Porengröße von etwa 0,3 Millimeter bis etwa 5 Millimeter hat.

19. Flexibles Substrat wie in Anspruch 1 definiert, worin das Aktivierungsmittel auf eine Aktivierungstemperatur von etwa 170 °C bis etwa 300 °C erwärmt ist, worin die Zugabemenge der Aktivkohlebeschichtung etwa 5 % bis etwa 50 % der Masse des Substrats beträgt, wobei das Substrat eine mittlere Porengröße von etwa 0,1 Millimeter bis etwa 10 Millimeter hat.

20. Flexibles Substrat wie in Anspruch 19 definiert, worin die verstreckbare Komponente ein elastomeres Polymer umfasst.

21. Flexibles Substrat wie in Anspruch 20 definiert, worin der Erweichungspunkt des elastomeren Polymers größer ist als die Aktivierungstemperatur.

22. Flexibles Substrat wie in Anspruch 19, 20 oder 21 definiert, worin das Substrat eine mittlere Porengröße von etwa 0,3 Millimeter bis etwa 5 Millimeter hat.

23. Flexibles Substrat wie in einem der vorhergehenden Ansprüche definiert, in welchem eine integrale Aktivkohlebeschichtung um die Fasern des Substrats herum gebildet ist.

24. Flexibles Substrat wie in einem der vorhergehenden Ansprüche definiert, in welchem die Beschichtung ein Netzwerk bildet, welches das Substratmaterial umgibt, und/oder chemische Bindungen zwischen der Beschichtung und dem Substratmaterial gebildet sind.

25. Flexibles Substrat wie in einem der vorhergehenden Ansprüche definiert, in welchem das polymere Material vernetzt ist.

26. Verfahren zum Bilden eines flexiblen Substrats, verwendet in einem absorbierenden Gegenstand (10) in Form einer Hygienebinde, Windel, Windelhose, Damenbinde oder Kindertrainingshose, **gekennzeichnet durch** Bereitstellen eines Substrats mit einer ersten Oberfläche und einer zweiten Oberfläche, worin das flexible Substrat eine verstreckbare Komponente enthält, die bei Anwendung einer Kraft auf eine gestreckte vorgespannte Länge verstreckbar ist, die wenigstens etwa 120 % ihrer ungestreckten Länge beträgt,
Aufbringen eines polymeren Materials und eines Aktivierungsmittels auf die erste Oberfläche des Substrats,
Erwärmen des polymeren Materials und des Aktivierungsmittels auf eine Aktivierungstemperatur von etwa 100 °C bis etwa 300 °C zum Bilden einer Aktivkohlebeschichtung und
Einarbeiten des Substrats in den absorbierenden Gegenstand.

27. Verfahren wie in Anspruch 26 definiert, worin die verstreckbare Komponente ein elastomeres Polymer umfasst.

28. Verfahren wie in Anspruch 27 definiert, worin der Erweichungspunkt des elastomeren Polymers größer ist als die Aktivierungstemperatur.

29. Verfahren wie in Anspruch 26, 27 oder 28 definiert, worin die verstreckbare Komponente ein polymeres Gitter enthält.

30. Verfahren wie in einem der Ansprüche 26 bis 29 definiert, worin das polymere Material ausgewählt ist aus der Gruppe bestehend aus Polyacrylnitril, phenolischen Harzen, Ethylen-Vinylacetat oder Copolymeren davon, Polyvinylalkohol, Cellulose oder anderen natürlichen oder synthetischen Polysacchariden, Cellulosederivaten oder anderen Polysaccharidderivaten, Polystyrol, Polypropylen, Polyvinylchlorid, Polymethacrylaten, Polymethacrylsäuren, Polymilchsäure und Kombinationen davon.

31. Verfahren wie in einem der Ansprüche 26 bis 30 definiert, worin das Aktivierungsmittel ausgewählt ist aus der Gruppe bestehend aus Säuren, Metallhalogeniden, Hydroxiden und Kombinationen davon.

32. Verfahren wie in einem der Ansprüche 26 bis 31 definiert, worin das polymere Material und das Aktivierungsmittel auf eine Temperatur von etwa 170 °C bis etwa 300 °C erwärmt werden.

33. Verfahren wie in einem der Ansprüche 26 bis 32 definiert, worin die Zugabemenge der Aktivkohlebeschichtung etwa 5 % bis etwa 50 % der Masse des Substrats beträgt.

34. Verfahren wie in einem der Ansprüche 26 bis 33 definiert, worin die Aktivkohlebeschichtung weniger als 100 % von der ersten Oberfläche des Substrats umfasst.

35. Verfahren wie in einem der Ansprüche 26 bis 33 definiert, worin das Substrat eine mittlere Porengröße von etwa 0,1 Millimeter bis etwa 10 Millimeter hat.

36. Verfahren wie in Anspruch 35 definiert, worin das Substrat eine mittlere Porengröße von etwa 0,3 Millimeter bis etwa 5 Millimeter hat.

37. Verfahren wie in einem der Ansprüche 26 bis 36 definiert, worin das polymere Material, das Aktivierungsmittel oder Kombinationen davon auf die erste Oberfläche des Substrats gedruckt werden.

38. Verfahren wie in einem der Ansprüche 26 bis 37 definiert, worin eine zweite Aktivkohlebeschichtung auf der zweiten Oberfläche des Substrats gebildet wird.

**Revendications**

1. Substrat flexible mis en oeuvre dans un article absorbant (10) sous la forme d'une serviette hygiénique, d'une couche, d'une couche-culotte, d'une serviette féminine ou d'une culotte d'apprentissage pour les enfants, **caractérisé en ce que** le substrat est appliqué avec un revêtement de charbon actif, ledit revêtement de charbon actif étant formé à partir d'une matière polymère et d'un agent d'activation chauffé jusqu'à une température d'activation d'environ 100°C à environ 300°C, où le substrat flexible contient un composant étirable qui, par l'application d'une force, est étirable jusqu'à une longueur en biais étirée qui est d'au moins environ 120 % de sa longueur non étirée.

2. Substrat flexible selon la revendication 1, dans lequel ledit composant étirable comprend un polymère élastomère.

3. Substrat flexible selon la revendication 2, dans lequel le point de ramollissement dudit polymère élastomère est supérieur à ladite température d'activation.

4. Substrat flexible selon la revendication 3, dans lequel ledit polymère élastomère est choisi parmi le groupe constitué de fluoropolymères ; de perfluoropolymères ; de polymères de nitrile hautement saturés ; de polymères d'éthylène/acétate de vinyle, de polymères siliconés ; d'élastomères de polyacrylates ; et de leurs combinaisons.

5. Substrat flexible selon la revendication 2, dans lequel ledit polymère élastomère est présent dans un stratifié élastique.

6. Substrat flexible selon la revendication 5, dans lequel ledit stratifié élastique est choisi parmi le groupe constitué de stratifiés collés par étranglement, de stratifiés collés par étirage, de stratifiés collés par étranglement/étirage et de leurs combinaisons.

7. Substrat flexible selon la revendication 1, dans lequel ledit composant étirable contient une maille polymère.

8. Substrat flexible selon la revendication 7, dans lequel ladite maille polymère est choisie parmi le groupe constitué de mailles de polyester, de mailles de nylon et de leurs combinaisons.

9. Substrat flexible selon l'une quelconque des revendications précédentes, dans lequel ladite matière polymère est choisie parmi le groupe constitué d'un polyacrylonitrile, de résines phénoliques, de l'éthylène/acétate de vinyle ou de leurs copolymères, d'un alcool polyvinylique, de la cellulose ou d'autres polysaccharides naturels ou synthétiques, de dérivés de la cellulose ou d'autres dérivés de polysaccharide, d'un polystyrène, d'un polypropylène, d'un chlorure de polyvinyle, de polyméthacrylates, d'acides polyméthacryliques, d'un acide polylactique et de leurs combinaisons.

10. Substrat flexible selon l'une quelconque des revendications précédentes, dans lequel ledit agent d'activation comprend un composé choisi parmi le groupe constitué d'acides, d'halogénures de métaux, d'hydroxydes et de leurs combinaisons.

11. Substrat flexible selon l'une quelconque des revendications précédentes, dans lequel ladite matière polymère et ledit agent d'activation sont chauffés jusqu'à une température d'environ 170°C à environ 300°C.

12. Substrat flexible selon l'une quelconque des revendications précédentes, où ledit substrat contient une étoffe non tissée.

13. Substrat flexible selon l'une quelconque des revendications précédentes, dans lequel le taux d'appoint dudit revêtement de charbon actif est d'environ 5 % à environ 50 % de la masse dudit substrat.

14. Substrat flexible selon l'une quelconque des revendications précédentes, dans lequel ledit revêtement de charbon actif est appliqué en un motif pré-choisi sur une première surface dudit substrat.

15. Substrat flexible selon l'une quelconque des revendications précédentes, dans lequel ledit revêtement de charbon actif constitue moins de 100 % d'au moins une surface dudit substrat.

16. Substrat flexible selon la revendication 15, dans lequel ledit revêtement de charbon actif constitue d'environ 20 % à environ 60 % d'au moins une surface dudit substrat.

**17.** Substrat flexible selon l'une quelconque des revendications précédentes, où ledit substrat présente une taille de pores moyenne d'environ 0,1 millimètre à environ 10 millimètres.

**18.** Substrat flexible selon la revendication 17, où ledit substrat présente une taille de pores moyenne d'environ 0,3 millimètre à environ 5 millimètres.

**19.** Substrat flexible selon la revendication 1, dans lequel l'agent d'activation est chauffé jusqu'à une température d'activation d'environ 170°C à environ 300°C, où le taux d'appoint dudit revêtement de charbon actif est d'environ 5 % à environ 50 % de la masse dudit substrat, ledit substrat présentant une taille de pores moyenne d'environ 0,1 millimètre à environ 10 millimètres.

**20.** Substrat flexible selon la revendication 19, dans lequel ledit composant étirable comprend un polymère élastomère.

**21.** Substrat flexible selon la revendication 20, dans lequel le point de ramollissement dudit polymère élastomère est supérieur à ladite température d'activation.

**22.** Substrat flexible selon la revendication 19, 20 ou 21, où ledit substrat présente une taille de pores moyenne d'environ 0,3 millimètre à environ 5 millimètres.

**23.** Substrat flexible selon l'une quelconque des revendications précédentes, dans lequel un revêtement intégral de charbon actif est formé autour des fibres du substrat.

**24.** Substrat flexible selon l'une quelconque des revendications précédentes, dans lequel le revêtement forme un réseau qui entoure la matière de substrat et/ou des liaisons chimiques sont formées entre le revêtement et la matière de substrat.

**25.** Substrat flexible selon l'une quelconque des revendications précédentes, dans lequel la matière polymère est réticulée.

**26.** Procédé de formation d'un substrat flexible utilisé dans un article absorbant (10) sous la forme d'une serviette hygiénique, d'une couche, d'une couche-culotte, d'une serviette féminine ou d'une culotte d'apprentissage pour les enfants, **caractérisé par** la mise à disposition d'un substrat présentant une première surface et une deuxième surface, où le substrat flexible contient un composant étirable qui, par l'application d'une force, est étirable jusqu'à une longueur en biais étirée qui est d'au moins 120 % de sa longueur non étirée ; l'application d'une matière polymère et d'un agent d'activation sur ladite première surface dudit substrat ; le chauffage de ladite matière polymère et dudit agent d'activation jusqu'à une température d'activation d'environ 100°C à environ 300°C pour former un revêtement de charbon actif ; et l'incorporation du substrat dans un dit article absorbant.

**27.** Procédé selon la revendication 26, dans lequel ledit composant étirable comprend un polymère élastomère.

**28.** Procédé selon la revendication 27, dans lequel le point de ramollissement dudit polymère élastomère est supérieur à ladite température d'activation.

**29.** Procédé selon la revendication 26, 27 ou 28, dans lequel ledit composant étirable contient une maille polymère.

**30.** Procédé selon l'une quelconque des revendications 26 à 29, dans lequel ladite matière polymère est choisie parmi le groupe constitué d'un polyacrylonitrile, de résines phénoliques, de l'éthylène/acétate de vinyle ou de leurs copolymères, d'un alcool polyvinylique, de la cellulose ou d'autres polysaccharides naturels ou synthétiques, de dérivés de la cellulose ou d'autres dérivés de polysaccharide, d'un polystyrène, d'un polypropylène, d'un chlorure de polyvinyle, de polyméthacrylates, d'acides polyméthacryliques, d'un acide polylactique et de leurs combinaisons.

**31.** Procédé selon l'une quelconque des revendications 26 à 30, dans lequel ledit agent d'activation est choisi parmi le groupe constitué d'acides, d'halogénures de métaux, d'hydroxydes et de leurs combinaisons.

**32.** Procédé selon l'une quelconque des revendications 26 à 31, dans lequel ladite matière polymère et ledit agent d'activation sont chauffés jusqu'à une température d'environ 170°C à environ 300°C.

**33.** Procédé selon l'une quelconque des revendications 26 à 32, dans lequel le taux d'appoint dudit revêtement de charbon actif est d'environ 5 % à environ 50 % de la masse dudit substrat.

**34.** Procédé selon l'une quelconque des revendications 26 à 33, dans lequel ledit revêtement de charbon actif constitue moins de 100 % de ladite première surface dudit substrat.

**35.** Procédé selon l'une quelconque des revendications 26 à 33, dans lequel ledit substrat présente une taille de pores moyenne d'environ 0,1 millimètre à environ 10 millimètres.

**36.** Procédé selon la revendication 35, dans lequel ledit substrat présente une taille de pores moyenne d'environ 0,3 millimètre à environ 5 millimètres.

**37.** Procédé selon l'une quelconque des revendications 26 à 36, dans lequel ladite matière polymère, ledit agent d'activation ou leurs combinaisons sont imprimés sur ladite première surface dudit substrat.

**38.** Procédé selon l'une quelconque des revendications 26 à 37, dans lequel un deuxième revêtement de charbon actif est formé sur ladite deuxième surface dudit substrat.

FIG. 1

**FIG. 2**

**FIG. 3**

**FIG. 4**

**FIG. 5**

120

142

144

148

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5834114 A
- US 3849241 A, Butin
- US 4340563 A, Appel
- US 3692618 A, Dorschner
- US 3802817 A, Matsuki
- US 3338992 A, Kinney
- US 3341394 A, Kinney
- US 3502763 A, Hartman
- US 3502538 A, Levy
- US 3542615 A, Dobo
- US 5382400 A, Pike
- US 4100324 A, Anderson
- US 5284703 A, Everhart
- US 5350624 A, Georger
- US 20010024716 A
- WO 0197972 A, Economy
- US 6362389 B, McDowall
- US 5226992 A
- US 4981747 A
- US 4965122 A
- US 5336545 A
- US 4720415 A, VanderWielen
- US 5385775 A, Wright
- US 4789699 A, Kieffer
- US 4781966 A, Taylor
- US 4657802 A, Morman
- US 4655760 A, Morman
- US 5114781 A
- US 5116662 A
- US 4810556 A, Kobayashi
- US 6197404 A, Varona
- US 6150002 A, Varona
- US 4215682 A, Kubik
- US 4375718 A, Wadsworth
- US 4592815 A, Nakao
- US 4874659 A, Ando
- US 5401446 A, Tsai
- US 5883026 A, Reader
- US 5908598 A, Rousseau
- US 6365088 A, Knight
- US 4801494 A, Datta
- US 4908026 A, Sukiennik
- US 4798603 A, Meyer
- US 5248309 A, Serbiak
- US 5197959 A, Buell
- US 5085654 A, Buell
- US 5634916 A, Lavon
- US 5569234 A, Buell
- US 5716349 A, Taylor
- US 4950264 A, Osborn
- US 5009653 A, Osborn
- US 5509914 A, Osborn
- US 5649916 A, DiPalma
- US 5267992 A, Van Tillburg
- US 4687478 A, Van Tillburg
- US 4285343 A, McNair
- US 4608047 A, Mattingly
- US 5342342 A, Kitaoka
- US 5190563 A, Herron
- US 5702378 A, Widlund
- US 5308346 A, Sneller
- US 6110158 A, Kielpikowski
- WO 9900093 A, Patterson